# EUROPEAN PATENT APPLICATION

(11) **EP 4 609 779 A1**
(43) Date of publication of application: **03.09.2025**
(21) Application number: 25157012.3
(22) Date of filing: 11.02.2025
(51) Int. Cl.: A61B 1/00, A61B 8/00

(54) **MEDICAL SUPPORT DEVICE, ENDOSCOPE SYSTEM, AND MEDICAL SUPPORT METHOD**

(30) Priority: 27.02.2024 JP 2024027770
(71) Applicant: FUJIFILM Corporation, Tokyo 106-8620 (JP)
(72) Inventor: KIMURA, Yuya, Kanagawa, 258-8538 (JP); OKADA, Yuto, Kanagawa, 258-8538 (JP)
(74) Representative: HGF

(57) **Abstract**

Provided are a medical support device, an endoscope system, and a medical support method capable of allowing a user to visually recognize information suitable for a type of an endoscope connected to an image processing device.

A medical support method includes acquiring a first optical image and an ultrasound image in a case in which an ultrasound endoscope is connected to an image processing device, acquiring a second optical image in a case in which an optical endoscope is connected to the image processing device, outputting the first optical image and the ultrasound image to a display device in a case in which the ultrasound endoscope is connected to the image processing device; and outputting the second optical image and a first recognition result, which is obtained by causing a trained model for an optical image to recognize a first feature region shown in the second optical image, to the display device in a case in which the optical endoscope is connected to the image processing device.

## Description

### BACKGROUND OF THE INVENTION

### 1. Field of the Invention

The present disclosure relates to a medical support device, an endoscope system, and a medical support method.

### 2. Description of the Related Art

JP2021-013690A discloses a display control device that displays an endoscopic image and an ultrasound image obtained by an endoscope on the same screen. The display control device described in JP2021-013690A comprises: an acquisition unit that acquires a determination result from a determination unit that determines whether a target of interest of an observer who observes an inside of a living body via a screen is an endoscopic image or an ultrasound image; and a controller that switches between a first state in which the screen displays the endoscopic image more preferentially than the ultrasound image and a second state in which the screen displays the ultrasound image more preferentially than the endoscopic image, based on the determination result acquired by the acquisition unit.

WO2020/075578A discloses a medical image processing device. The medical image processing device described in WO2020/075578A comprises: an image acquisition unit that acquires a medical image; a determination unit that determines an illumination mode in a case in which the medical image is captured; a recognition unit that performs first recognition for the medical image in a case in which it is determined that the illumination mode is a first illumination mode and that performs second recognition for the medical image in a case in which it is determined that the illumination mode is a second illumination mode; and a display controller that causes a display device to perform first display in accordance with a result of the first recognition in a case in which it is determined that the illumination mode is the first illumination mode, and that causes the display device to perform second display in accordance with a result of the second recognition in a case in which it is determined that the illumination mode is the second illumination mode. The recognition unit includes a first recognizer that is configured by learning, that performs first recognition, and that detects a region of interest from the medical image, and a second recognizer that is configured by learning, that performs second recognition, and that classifies the medical image.

### SUMMARY OF THE INVENTION

One embodiment according to the present disclosure provides a medical support device, an endoscope system, and a medical support method capable of allowing a user to visually recognize information suitable for a type of an endoscope connected to an image processing device.

A first aspect according to the present disclosure provides a medical support device comprising: a processor, in which the processor is configured to: acquire first image information including a first optical image and an ultrasound image, which are generated by performing imaging via an ultrasound endoscope, in a case in which the ultrasound endoscope is connected to an image processing device to which the ultrasound endoscope and an optical endoscope are connectable; acquire second image information including a second optical image, which is generated by performing imaging via the optical endoscope, in a case in which the optical endoscope is connected to the image processing device; output the first optical image and the ultrasound image, which are included in the first image information, to a display device in a case in which the ultrasound endoscope is connected to the image processing device; and output the second optical image, which is included in the second image information, and a first recognition result, which is obtained by inputting the second optical image to a trained model for an optical image and causing the trained model for an optical image to recognize a first feature region shown in the second optical image, to the display device in a case in which the optical endoscope is connected to the image processing device.

A second aspect according to the present disclosure provides the medical support device according to the first aspect, in which the medical support device and the image processing device are separate bodies.

A third aspect according to the present disclosure provides the medical support device according to the first or second aspect, in which the medical support device is connected to the image processing device by using a cable, and the processor is configured to acquire the first image information and the second image information from the image processing device via the cable.

A fourth aspect according to the present disclosure provides the medical support device according to any one of the first to third aspects, in which the image processing device includes a first image processing device and a second image processing device, the first image processing device generates the ultrasound image based on a reflected wave of ultrasound emitted from the ultrasound endoscope, and the second image processing device generates the first optical image based on a first image signal obtained by performing optical imaging via the ultrasound endoscope and generates the second optical image based on a second image signal obtained by performing optical imaging via the optical endoscope.

A fifth aspect according to the present disclosure provides the medical support device according to the fourth aspect, in which the first image information is generated by the first image processing device or the second image processing device, the second image information is generated by the first image processing device or the second image processing device, and the processor is configured to: acquire the first image information from the first image processing device or the second image processing device; and acquire the second image information from the first image processing device or the second image processing device.

A sixth aspect according to the present disclosure provides the medical support device according to the fourth or fifth aspect, in which the first image processing device, the second image processing device, and the medical support device are separate bodies.

A seventh aspect according to the present disclosure provides the medical support device according to any one of the first to sixth aspects, in which each of the first image information and the second image information includes first specification information and/or second specification information, the first specification information is information for specifying a type of an endoscope connected to the image processing device, the second specification information is information for specifying a type of an image included in the first image information or the second image information, the types of the endoscope are the ultrasound endoscope and the optical endoscope, the types of the image are the ultrasound image, the first optical image, and the second optical image, and the processor is configured to specify whether the ultrasound endoscope is connected to the image processing device or the optical endoscope is connected to the image processing device, based on the first specification information and/or the second specification information.

An eighth aspect according to the present disclosure provides the medical support device according to any one of the first to seventh aspects, in which the processor is configured to specify whether the ultrasound endoscope is connected to the image processing device or the optical endoscope is connected to the image processing device, by analyzing an image included in the first image information or the second image information.

A ninth aspect according to the present disclosure provides the medical support device according to any one of the first to eighth aspects, in which the processor is configured to not output information, which is obtained from the trained model for an optical image by inputting the first optical image included in the first image information to the trained model for an optical image, to the display device in a case in which the ultrasound endoscope is connected to the image processing device.

A tenth aspect according to the present disclosure provides the medical support device according to any one of the first to ninth aspects, in which the processor is configured to not output information, which is obtained from the trained model for an optical image by inputting the first optical image included in the first image information to the trained model for an optical image, to the display device in a case in which the ultrasound image is displayed in a first display region of the display device.

An eleventh aspect according to the present disclosure provides the medical support device according to the ninth or tenth aspect, in which the trained model for an optical image is a trained model obtained by performing machine learning for the second optical image.

A twelfth aspect according to the present disclosure provides the medical support device according to any one of the first to eleventh aspects, in which the first recognition result includes first positional information for specifying a position of the first feature region, first feature information for specifying a medical feature of the first feature region, and/or first part information for specifying a part inside a body as a target of an examination via the optical endoscope.

A thirteenth aspect according to the present disclosure provides the medical support device according to the twelfth aspect, in which the trained model for an optical image includes a first trained model to which the second optical image is input, and the first trained model generates the first positional information based on the input second optical image.

A fourteenth aspect according to the present disclosure provides the medical support device according to any one of the first to thirteenth aspects, in which the processor is configured to output a second recognition result, which is obtained by inputting the ultrasound image included in the first image information to a trained model for an ultrasound image and causing the trained model for an ultrasound image to recognize a second feature region shown in the ultrasound image, to the display device in a case in which the ultrasound image is displayed in a first display region of the display device.

A fifteenth aspect according to the present disclosure provides the medical support device according to the fourteenth aspect, in which the second recognition result includes second positional information for specifying a position of the second feature region, second feature information for specifying a medical feature of the second feature region, and/or second part information for specifying a part inside a body as a target of an examination via the ultrasound endoscope.

A sixteenth aspect according to the present disclosure provides the medical support device according to the fifteenth aspect, in which the trained model for an ultrasound image includes a second trained model to which the ultrasound image is input, and the second trained model generates the second positional information based on the input ultrasound image.

A seventeenth aspect according to the present disclosure provides an endoscope system comprising: the medical support device according to any one of the first to sixteenth aspects; and the image processing device including a first image processing device and a second image processing device, in which the first image processing device generates the ultrasound image based on a reflected wave of ultrasound emitted from the ultrasound endoscope, and the second image processing device generates the first optical image based on a first image signal obtained by performing optical imaging via the ultrasound endoscope and generates the second optical image based on a second image signal obtained by performing optical imaging via the optical endoscope.

An eighteenth aspect according to the present disclosure provides a medical support method comprising: acquiring first image information including a first optical image and an ultrasound image, which are generated by performing imaging via an ultrasound endoscope, in a case in which the ultrasound endoscope is connected to an image processing device to which the ultrasound endoscope and an optical endoscope are connectable; acquiring second image information including a second optical image, which is generated by performing imaging via the optical endoscope, in a case in which the optical endoscope is connected to the image processing device; outputting the first optical image and the ultrasound image, which are included in the first image information, to a display device in a case in which the ultrasound endoscope is connected to the image processing device; and outputting the second optical image, which is included in the second image information, and a first recognition result, which is obtained by inputting the second optical image to a trained model for an optical image and causing the trained model for an optical image to recognize a first feature region shown in the second optical image, to the display device in a case in which the optical endoscope is connected to the image processing device.

### BRIEF DESCRIPTION OF THE DRAWINGS

Fig. 1 is a conceptual diagram showing an example of an aspect in which a medical support system is used by a doctor.
Fig. 2 is a conceptual diagram showing an example of an overall configuration of the medical support system in a case in which an ultrasound endoscope is used.
Fig. 3 is a conceptual diagram showing an example of an overall configuration of the medical support system in a case in which an optical endoscope is used.
Fig. 4 is a block diagram showing an example of a hardware configuration of the medical support system.
Fig. 5 is a block diagram showing an example of a hardware configuration of a function expansion device included in the medical support system.
Fig. 6 is a conceptual diagram showing an example of an aspect in which ultrasound image information is generated by an ultrasound endoscope processing device and then output to an optical endoscope processing device.
Fig. 7 is a conceptual diagram showing an example of an aspect in which ultrasound endoscopic image information is generated by the optical endoscope processing device and then output to the function expansion device.
Fig. 8 is a conceptual diagram showing an example of an aspect in which optical endoscopic image information is generated by the optical endoscope processing device and then output to the function expansion device.
Fig. 9 is a conceptual diagram showing an example of a processing content of the function expansion device in a case in which the ultrasound image and an ultrasound image recognition result are displayed on a screen in accordance with first layout information.
Fig. 10 is a conceptual diagram showing an example of a processing content of the function expansion device in a case in which a first optical image is displayed on the screen in accordance with the first layout information.
Fig. 11 is a conceptual diagram showing an example of a processing content of the function expansion device in a case in which a second optical image and an optical image recognition result are displayed on the screen in accordance with second layout information.
Fig. 12 is a flowchart showing an example of a flow of a medical support process.
Fig. 13 is a modification example of the ultrasound endoscopic image information.
Fig. 14 is a modification example of the optical endoscopic image information.
Fig. 15 is a conceptual diagram showing an example of an aspect in which the ultrasound endoscopic image information is generated by the ultrasound endoscope processing device and then output to the function expansion device.
Fig. 16 is a conceptual diagram showing an example of a processing content in a case in which a plurality of trained models are included in a second recognition model.
Fig. 17 is a conceptual diagram showing an example of a processing content in a case in which a plurality of trained models are included in a first recognition model.
Fig. 18 is a block diagram showing an example of a hardware configuration of the function expansion device in a case in which a third recognition model is stored in a storage of the function expansion device included in the medical support system.
Fig. 19 is a conceptual diagram showing an example of a processing content in a case in which recognition processing of an AI method is performed on the first optical image.
Fig. 20 is a conceptual diagram showing an example of a content of AI processing in a case in which a type of an endoscope connected to the image processing device is specified by analyzing an image included in the ultrasound endoscopic image information and an image included in the optical endoscopic image information.
Fig. 21 is a conceptual diagram showing an example of the series of processing in which a processor included in a computer issues a processing execution request to an external device via a network, the external device executes processing in accordance with the processing execution request, and the processor included in the computer receives a processing result from the external device.

### DESCRIPTION OF THE PREFERRED EMBODIMENTS

Hereinafter, examples of embodiments of a medical support device, an endoscope system, and a medical support method according to the present disclosure will be described with reference to the accompanying drawings.

First, the terms used in the following description will be described.

CPU is an abbreviation for "central processing unit". GPU is an abbreviation for "graphics processing unit". GPGPU is an abbreviation for "general-purpose computing on graphics processing units". APU is an abbreviation for "accelerated processing unit". TPU is an abbreviation for "tensor processing unit". RAM is an abbreviation for "random-access memory". NVM is an abbreviation for "non-volatile memory". EEPROM is an abbreviation for "electrically erasable programmable read-only memory". ASIC is an abbreviation for "application-specific integrated circuit". PLD is an abbreviation for "programmable logic device". FPGA is an abbreviation for "field-programmable gate array". SoC is an abbreviation for "system-on-a-chip". SSD is an abbreviation for "solid-state drive". USB is an abbreviation for "Universal Serial Bus". HDD is an abbreviation for "hard disk drive". EL is an abbreviation for "electro-luminescence". CMOS is an abbreviation for "complementary metal-oxide-semiconductor". CCD is an abbreviation for "charge-coupled device". AI is an abbreviation for "artificial intelligence". BLI is an abbreviation for "blue light imaging". LCI is an abbreviation for "linked color imaging". I/F is an abbreviation for "interface". LAN is an abbreviation for "local area network". WAN is an abbreviation for "wide area network". 5G is an abbreviation for "5th generation mobile communication system". IC is an abbreviation for "integrated circuit".

In the following description, a processor with a reference numeral (hereinafter, simply referred to as "processor") may be one physical or virtual operation device or a combination of a plurality of physical or virtual operation devices. Further, the processor may be one type of operation device or a combination of a plurality of types of operation devices. Examples of the operation device include a CPU, a GPU, a GPGPU, an APU, and a TPU.

In the following description, a memory with a reference numeral is a memory such as a RAM that temporarily stores information, and is used as a work memory by the processor.

In the following description, a storage with a reference numeral is one or more non-volatile storage devices that store various programs, various parameters, and the like. Examples of the non-volatile storage device include a flash memory, a magnetic disk, and a magnetic tape. Examples of the storage also include a cloud storage.

In the following embodiment, an external I/F with a reference numeral controls the transmission and the reception of various types of information among a plurality of devices connected to each other. Examples of the external I/F include a USB interface. A communication I/F including a communication processor, an antenna, and the like may be applied to the external I/F. The communication I/F controls communication among a plurality of computers. Examples of a communication standard applied to the communication I/F include a wireless communication standard including 5G, Wi-Fi (registered trademark), or Bluetooth (registered trademark).

In the following embodiment, "A and/or B" is synonymous with "at least one of A or B". That is, "A and/or B" may mean only A, only B, or a combination of A and B. In the present specification, the same concept as "A and/or B" also applies to a case in which three or more matters are expressed by association with "and/or".

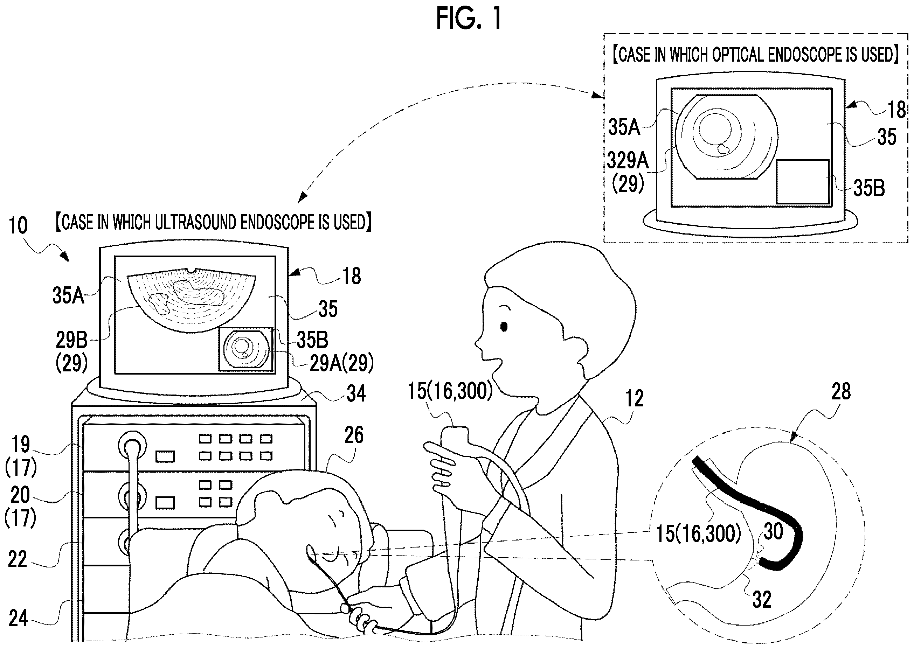

Fig. 1 is a conceptual diagram showing an example of an aspect in which an endoscope system 10 is used. The endoscope system 10 is used by a user 12 in endoscopy or the like. The user 12 is, for example, a doctor.

The endoscope system 10 comprises an endoscope 15, an image processing device 17, a display device 18, a light source device 22, and a function expansion device 24. The endoscope system 10 in the present embodiment is an example of an "endoscope system" according to the present disclosure. In addition, the image processing device 17 in the present embodiment is an example of an "image processing device" according to the present disclosure. In addition, the display device 18 in the present embodiment is an example of a "display device" according to the present disclosure. In addition, the function expansion device 24 in the present embodiment is an example of a "medical support device" according to the present disclosure.

The endoscope 15 is inserted into a lumen organ of a subject 26 (for example, a patient) and is operated by the user 12 to perform a medical treatment in the lumen organ. In the example shown in Fig. 1, an upper gastrointestinal tract 28 is shown as an example of the lumen organ. The medical treatment performed by using the endoscope 15 inserted into the upper gastrointestinal tract 28 includes observation and treatment. Examples of a target on which the medical treatment is performed include an esophagus, a stomach, a duodenum, a bile duct, a gallbladder, a pancreatic duct, and a pancreas.

The endoscope 15 performs imaging in the upper gastrointestinal tract 28. Here, the concept of the imaging includes optical imaging and ultrasound imaging. In other words, the imaging described herein refers to processing of detecting physical energy (for example, reflected light or reflected waves of ultrasound) from an observation target in the upper gastrointestinal tract 28 and outputting a detection result as an electric signal that can be imaged.

The image processing device 17 is a device to which the endoscope 15 can be connected, acquires an electric signal from the endoscope 15, and performs various types of processing on the acquired electric signal to form an image of the electric signal. That is, the image processing device 17 generates a medical image 29 based on the electric signal acquired from the endoscope 15. The medical image 29 is displayed on the display device 18. Generally, the medical image 29 is also referred to as an endoscopic image.

As a type of the endoscope 15, there are two types of an ultrasound endoscope 16 and an optical endoscope 300. The ultrasound endoscope 16 is a hybrid endoscope of an optical upper endoscope and an ultrasound upper endoscope (that is, the endoscope in which an optical upper endoscope and the ultrasound upper endoscope are combined). The optical upper endoscope is, for example, inserted into the upper gastrointestinal tract 28, emits light 30 in the upper gastrointestinal tract 28, detects reflected light obtained by being reflected by an interior wall 32 of the upper gastrointestinal tract 28, and outputs a detection result to the image processing device 17 as the electric signal. The ultrasound upper endoscope is, for example, inserted into the upper gastrointestinal tract 28, detects the reflected wave obtained by emitting ultrasound in the upper gastrointestinal tract 28, and outputs a detection result to the image processing device 17 as the electric signal. The optical endoscope 300 is the optical upper endoscope. It should be noted that, here, although the ultrasound endoscope 16 is described, this is merely an example, and the present disclosure is established even in a case in which an ultrasound endoscope in which an optical endoscope and an ultrasound endoscope are separate bodies is used. A first example of the ultrasound endoscope in which the optical endoscope and the ultrasound endoscope are separate bodies is an ultrasound endoscope used by inserting a thin ultrasound probe into a forceps port of the optical endoscope. A second example of the ultrasound endoscope in which the optical endoscope and the ultrasound endoscope are separate bodies is an ultrasound endoscope in which an ultrasound probe is externally attached to a distal end of the optical endoscope.

Here, for convenience of description, the upper endoscope used for the upper endoscopy is described, but this is merely an example, and the present disclosure is established even in a case of a lower endoscope used for lower endoscopy. Further, the present disclosure is established even in a case of an endoscope (for example, a bronchoscope, an endoscope for otorhinolaryngology, an intracranial endoscope, or a laparoscope) used for endoscopy of an organ other than the digestive organ.

The image processing device 17 includes an ultrasound endoscope processing device 19 and an optical endoscope processing device 20. The ultrasound endoscope 16 and the optical endoscope 300 are selectively connected to the optical endoscope processing device 20. In a case in which the ultrasound endoscope 16 is connected to the optical endoscope processing device 20, the optical endoscope processing device 20 generates a first optical image 29A as one of the medical images 29 based on the reflected light detected by the ultrasound endoscope 16. In addition, in a case in which the ultrasound endoscope 16 is connected to the ultrasound endoscope processing device 19, the ultrasound endoscope processing device 19 generates an ultrasound image 29B as one of the medical images 29 based on the reflected wave detected by the ultrasound endoscope 16. In addition, in a case in which the optical endoscope 300 is connected to the optical endoscope processing device 20, the optical endoscope processing device 20 generates a second optical image 329A as one of the medical images 29 based on the reflected light detected by the optical endoscope 300.

The ultrasound endoscope processing device 19 in the present embodiment is an example of a "first image processing device" according to the present disclosure. In addition, the optical endoscope processing device 20 in the present embodiment is an example of a "second image processing device" according to the present disclosure. In addition, the first optical image 29A in the present embodiment is an example of a "first optical image" according to the present disclosure. In addition, the second optical image 329A in the present embodiment is an example of a "second optical image" according to the present disclosure. In addition, the ultrasound image 29B in the present embodiment is an example of an "ultrasound image" according to the present disclosure.

The ultrasound endoscope processing device 19, the optical endoscope processing device 20, the light source device 22, and the function expansion device 24 are installed in a wagon 34. A plurality of tables are provided in the wagon 34 along an up-down direction, and the function expansion device 24, the light source device 22, the optical endoscope processing device 20, and the ultrasound endoscope processing device 19 are installed from a table on a lower stage side to a table on an upper stage side. In addition, the display device 18 is installed on the uppermost stage in the wagon 34.

The display device 18 displays various types of information including the image. Examples of the display device 18 include a liquid-crystal display and an EL display. In addition, a tablet terminal with a display may be used instead of or together with the display device 18.

A screen 35 is displayed on the display device 18. The screen 35 includes a plurality of display regions. In the example shown in Fig. 1, a first display region 35A and a second display region 35B are shown as examples of the plurality of display regions. A size of the first display region 35A is larger than a size of the second display region 35B. The first display region 35A is used as a main display region, and the second display region 35B is used as a sub-display region. A size relationship between the first display region 35A and the second display region 35B is not limited to this, and the size relationship between the first display region 35A and the second display region 35B need only be in a size relationship that fits in the screen 35, and the size relationship between the first display region 35A and the second display region 35B may be fixed or variable.

In a case in which the ultrasound endoscope 16 is used by the user 12, the ultrasound endoscope 16 is connected to the ultrasound endoscope processing device 19 and the optical endoscope processing device 20. In a case in which the ultrasound endoscope 16 is connected to the ultrasound endoscope processing device 19 and the optical endoscope processing device 20, the ultrasound image 29B is displayed in the first display region 35A, and the first optical image 29A is displayed in the second display region 35B.

On the other hand, in a case in which the optical endoscope 300 is used by the user 12, the optical endoscope 300 is connected to the optical endoscope processing device 20. In a case in which the optical endoscope 300 is connected to the optical endoscope processing device 20, the second optical image 329A is displayed in the first display region 35A.

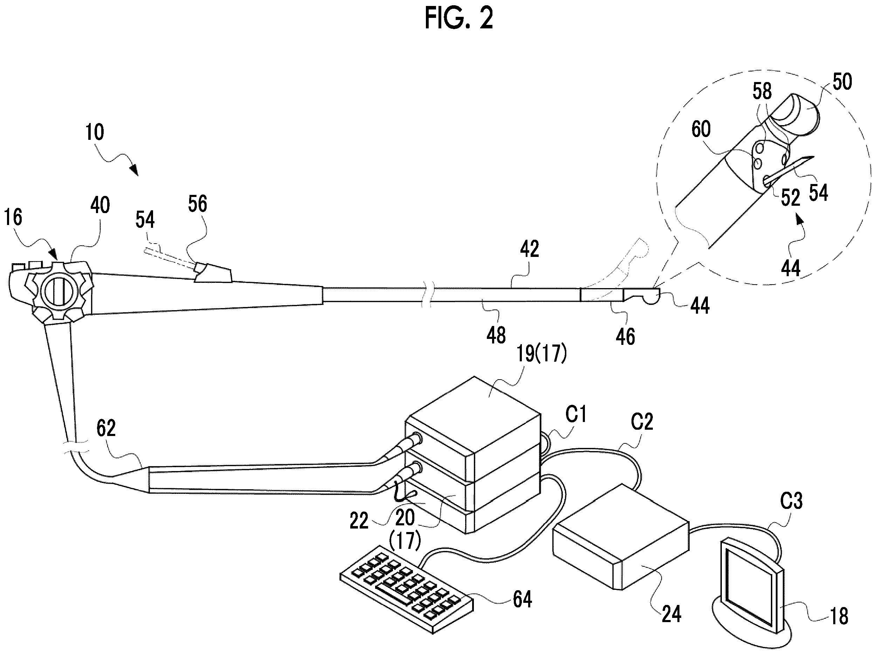

Fig. 2 is a conceptual diagram showing an example of an overall configuration of the endoscope system 10 in a case in which the ultrasound endoscope 16 is used. As shown in Fig. 2, the ultrasound endoscope 16 comprises an operating part 40 and an insertion part 42. The insertion part 42 is formed in a tubular shape. The insertion part 42 includes a distal end part 44, a bendable part 46, and a soft part 48. The distal end part 44, the bendable part 46, and the soft part 48 are disposed in an order of the distal end part 44, the bendable part 46, and the soft part 48 from a distal end side to a base end side of the insertion part 42. The soft part 48 is formed of a material having a long and flexible shape and connects the operating part 40 and the bendable part 46. The bendable part 46 is partially bent or rotated about an axial center of the insertion part 42 by operating the operating part 40. The insertion part 42 is fed to the back side of the upper gastrointestinal tract 28 while being bent in accordance with a shape of the lumen organ (for example, a shape of a pathway of the upper gastrointestinal tract 28) or being rotated about an axis of the insertion part 42.

The distal end part 44 is provided with an ultrasound probe 50 and a treatment tool opening 52. The ultrasound probe 50 is provided on a distal end side of the distal end part 44. The ultrasound probe 50 is a convex type ultrasound probe that emits the ultrasound to receive the reflected wave obtained by reflecting the emitted ultrasound from the emission target region (for example, an organ such as a pancreas). Here, the convex type ultrasound probe is described as an example of the ultrasound probe 50, but this is merely an example, and, for example, a radial type ultrasound probe may also be used.

The treatment tool opening 52 is formed on a base end side of the distal end part 44 with respect to the ultrasound probe 50. The treatment tool opening 52 is an opening for allowing a treatment tool 54 to protrude from the distal end part 44. A treatment tool insertion port 56 is formed at the operating part 40, and the treatment tool 54 is inserted into the insertion part 42 through the treatment tool insertion port 56. The treatment tool 54 passes through the insertion part 42 and then protrudes from the treatment tool opening 52 to the outside of the ultrasound endoscope 16. In addition, the treatment tool opening 52 is also used as a suction port for suctioning blood, body waste, and the like and as a delivery port for sending out a fluid.

In the example shown in Fig. 2, a puncture needle is shown as the treatment tool 54. It should be noted that this is merely an example, and the treatment tool 54 may be a grasping forceps, a papillotomy knife, a snare, a catheter, a guide wire, a cannula, a puncture needle with a guide sheath, and the like.

The distal end part 44 is provided with an illumination device 58 and a camera 60. The illumination device 58 emits the light 30 (see Fig. 1). Examples of the types of the light 30 emitted from the illumination device 58 include white light and special light. Examples of the special light include light for BLI and/or light for LCI.

The camera 60 is mounted in the ultrasound endoscope 16. The camera 60 images an observation target region in a state in which the camera 60 is inserted into the upper gastrointestinal tract 28 of the subject 26 and the light 30 is emitted by the illumination device 58 in the upper gastrointestinal tract 28. Examples of the camera 60 include a camera equipped with a CMOS image sensor. The CMOS image sensor is merely an example, and a camera equipped with another type of image sensor, such as a CCD image sensor, may be used.

The ultrasound endoscope 16 is connected to the image processing device 17 and the light source device 22. A housing of the image processing device 17 and a housing of the function expansion device 24 are physically separate from each other. That is, the image processing device 17 and the function expansion device 24 are separate bodies. The function expansion device 24 is connected to the image processing device 17.

A housing of the ultrasound endoscope processing device 19 and a housing of the optical endoscope processing device 20 are physically separate from each other. That is, the ultrasound endoscope processing device 19 and the optical endoscope processing device 20 are separate bodies. It should be noted that, here, the configuration in which the ultrasound endoscope processing device 19 and the optical endoscope processing device 20 are separate bodies is merely an example, and the ultrasound endoscope processing device 19 and the optical endoscope processing device 20 may be integrated with each other.

The ultrasound endoscope 16 is used in a state of being connected to the ultrasound endoscope processing device 19, the optical endoscope processing device 20, and the light source device 22 via a universal cord 62. The ultrasound endoscope processing device 19 is connected to the optical endoscope processing device 20 by using a cable C1, and the optical endoscope processing device 20 is connected to the function expansion device 24 by using a cable C2. That is, the image processing device 17 is connected to the function expansion device 24 by using the cables C1 and C2. The cables C1 and C2 in the present embodiment are examples of a "cable" according to the present disclosure. It should be noted that, in the present embodiment, although the wired connection is described, the present disclosure is established even in a case of wireless connection.

A reception device 64 is connected to the optical endoscope processing device 20. Further, the display device 18 is also connected to the function expansion device 24. That is, the image processing device 17 is connected to the display device 18 via the function expansion device 24.

The reception device 64 receives an instruction from the user 12 and outputs the received instruction to the optical endoscope processing device 20 as the electric signal. Examples of the reception device 64 include a keyboard, a mouse, a touch panel, a foot switch, a microphone, and/or a remote operation device.

the optical endoscope processing device 20 controls the light source device 22, transmits and receives various signals to and from the ultrasound endoscope 16, or transmits and receives various signals to and from the function expansion device 24, in accordance with the instruction received by the reception device 64.

The light source device 22 emits the light under the control of the optical endoscope processing device 20 to supply the light 30 (see Fig. 1) to the illumination device 58. The illumination device 58 is provided with a built-in light guide, and the light 30 supplied from the light source device 22 is emitted from the distal end part 44 via the light guide. The optical endoscope processing device 20 causes the camera 60 to perform the imaging, in accordance with the instruction received by the reception device 64, generates the first optical image 29A (see Fig. 1) based on the electric signal acquired from the camera 60, and outputs the first optical image 29A to the function expansion device 24. In addition, the ultrasound endoscope processing device 19 emits the ultrasound to the ultrasound probe 50 in accordance with the instruction received by the reception device 64, and generates the ultrasound image 29B (see Fig. 1) based on the reflected wave received by the ultrasound probe 50. The ultrasound endoscope processing device 19 outputs the generated ultrasound image 29B to the function expansion device 24.

The function expansion device 24 is a device that expands functions of the image processing device 17. The function expansion device 24 supports the medical treatment performed by using the endoscope 15 by executing processing using the medical image 29 input from the optical endoscope processing device 20. Examples of the processing using the medical image 29 include recognition processing of the AI method on the medical image 29. As described above, in a case in which the function expansion device 24 is a device that performs the recognition processing of the AI method on the medical image 29, even in a case in which the function of performing the recognition processing of the AI method on the medical image 29 is not mounted in the image processing device 17, the function of performing the recognition processing of the AI method on the medical image 29 is added as the function of the image processing device 17 by attaching the function expansion device 24 to the image processing device 17, so that the function of the image processing device 17 is expanded. The function expansion device 24 outputs various types of information including the medical image 29 input from the optical endoscope processing device 20 to the display device 18.

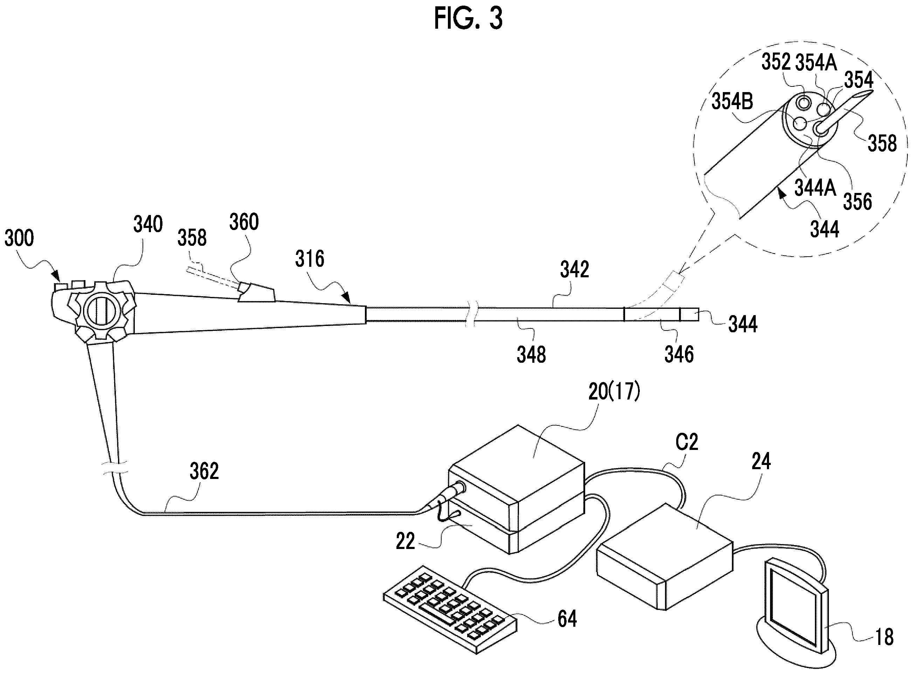

Fig. 3 is a conceptual diagram showing an example of an overall configuration of the endoscope system 10 in a case in which the optical endoscope 300 is used. As shown in Fig. 3, the optical endoscope 300 comprises an operating part 340 and an insertion part 342. The insertion part 342 is formed in a tubular shape. The insertion part 342 includes a distal end part 344, a bendable part 346, and a soft part 348. The distal end part 344, the bendable part 346, and the soft part 348 are disposed in an order of the distal end part 344, the bendable part 346, and the soft part 348 from a distal end side to a base end side of the insertion part 342. The soft part 348 is formed of a material having a long and flexible shape and connects the operating part 340 and the bendable part 346. The bendable part 346 is partially bent or rotated about an axial center of the insertion part 342 by operating the operating part 340. The insertion part 342 is fed to the back side of the upper gastrointestinal tract 28 while being bent in accordance with a shape of the lumen organ (for example, a shape of a pathway of the upper gastrointestinal tract 28) or being rotated about an axis of the insertion part 342.

The distal end part 344 is provided with a camera 352, an illumination device 354, and a treatment tool opening 356. The camera 352 and the illumination device 354 are provided on a distal end surface 344A of the distal end part 344. It should be noted that, here, although the form example is described in which the camera 352 and the illumination device 354 are provided on the distal end surface 344A of the distal end part 344, this is merely an example, and the optical endoscope 300 may be configured as a side-view endoscope by providing the camera 352 and the illumination device 354 on a side surface of the distal end part 344.

A fundamental configuration of the camera 352 is the same as the configuration of the camera 60 (see Fig. 2), but the camera 352 is a camera having higher performance than the camera 60. The image obtained by performing the imaging via the camera 352 is an image having a higher image quality than the image obtained by performing the imaging via the camera 60.

The illumination device 354 includes illumination windows 354A and 354B. The illumination device 354 emits the light 30 (see Fig. 1) through the illumination windows 354A and 354B. Examples of the light 30 emitted from the illumination device 354 include visible light (for example, white light) and invisible light (for example, near-infrared light). The illumination device 354 emits special light through the illumination windows 354A and 354B. Examples of the special light include light for BLI and/or light for LCI. The camera 352 images the inside of the upper gastrointestinal tract 28 via an optical method in a state in which the light 30 is emitted by the illumination device 354 inside the upper gastrointestinal tract 28.

The treatment tool opening 356 is an opening for allowing a treatment tool 358 to protrude from the distal end part 344. In addition, the treatment tool opening 356 is also used as a suction port for suctioning blood, body waste, and the like and as a delivery port for sending out a fluid.

A treatment tool insertion port 360 is formed at the operating part 340, and the treatment tool 358 is inserted into the insertion part 342 through the treatment tool insertion port 360. The treatment tool 358 passes through the insertion part 342 and protrudes from the treatment tool opening 356 to the outside. In the example shown in Fig. 3, an aspect is shown in which a puncture needle as the treatment tool 358 protrudes from the treatment tool opening 356. Here, the puncture needle is shown as the treatment tool 358, but this is merely an example, and the treatment tool 358 may be a grasping forceps, a papillotomy knife, a snare, a catheter, a guide wire, a cannula, and/or a puncture needle with a guide sheath.

The optical endoscope 300 is used in a state of being connected to the optical endoscope processing device 20 and the light source device 22 via a universal cord 362.

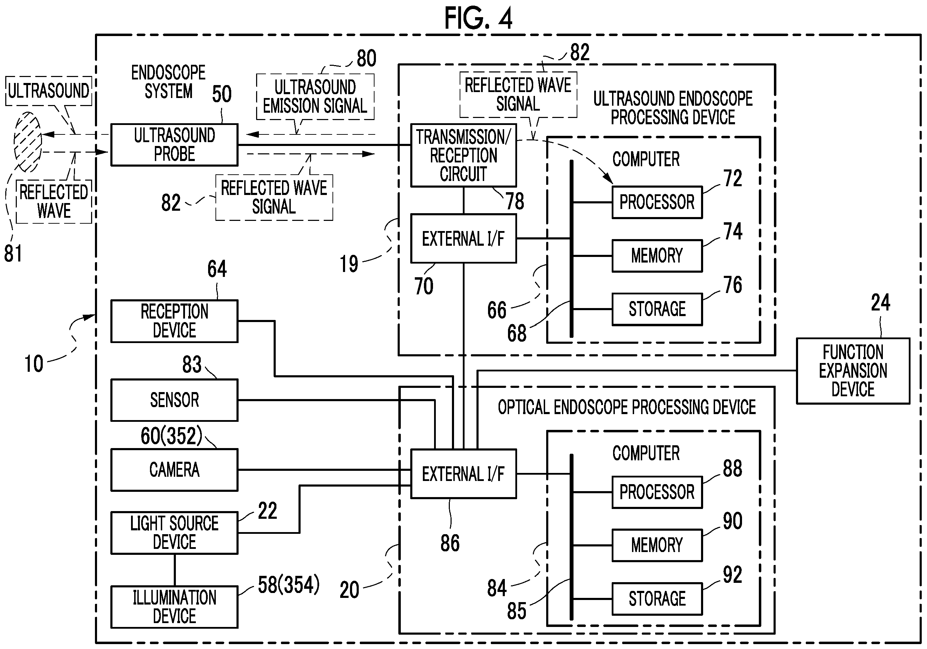

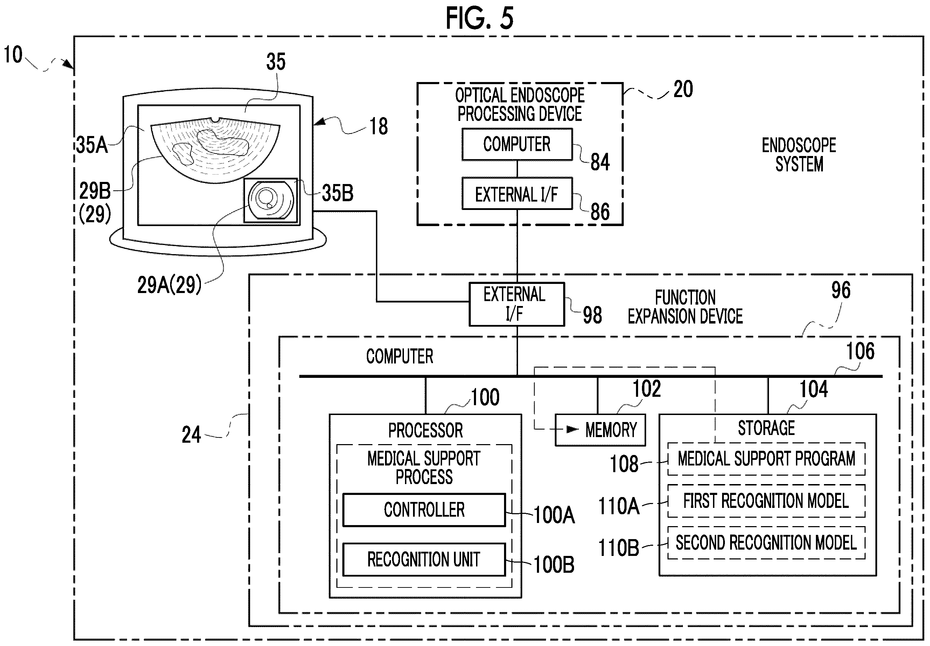

Figs. 4 and 5 are block diagrams showing an example of a hardware configuration of an electric system of the endoscope system 10. As shown in Fig. 4, the ultrasound endoscope processing device 19 comprises a computer 66, a bus 68, and an external I/F 70. The computer 66 comprises a processor 72, a memory 74, and a storage 76. The processor 72, the memory 74, the storage 76, and the external I/F 70 are connected to a bus 68. The processor 72 controls the entire ultrasound endoscope processing device 19. The memory 74 and the storage 76 are used by the processor 72.

The external I/F 70 controls the transmission and the reception of various types of information between one or more devices (hereinafter, also referred to as a "first external device") that are present outside the ultrasound endoscope processing device 19 and the processor 72.

A transmission/reception circuit 78 is connected to the external I/F 70, as one of the first external devices. In a case in which the ultrasound endoscope 16 is connected to the ultrasound endoscope processing device 19, the ultrasound probe 50 is connected to the transmission/reception circuit 78. The transmission/reception circuit 78 generates an ultrasound emission signal 80 having a pulse waveform to output the ultrasound emission signal 80 to the ultrasound probe 50 in accordance with an instruction from the processor 72. The ultrasound probe 50 converts the ultrasound emission signal 80 input from the transmission/reception circuit 78 into the ultrasound and emits the ultrasound to an emission target region 81 (for example, an organ such as a pancreas). The ultrasound probe 50 receives the reflected wave obtained by reflecting the ultrasound wave emitted from the ultrasound probe 50 from the emission target region 81, converts the reflected wave into a reflected wave signal 82, which is the electric signal, and outputs the reflected wave signal 82 to the transmission/reception circuit 78. The transmission/reception circuit 78 digitizes the reflected wave signal 82 input from the ultrasound probe 50 and outputs the digitized reflected wave signal 82 to the processor 72 via the external I/F 70. The processor 72 generates the ultrasound image 29B (see Fig. 1) showing an aspect of the emission target region 81 based on the reflected wave signal 82 input from the transmission/reception circuit 78 via the external I/F 70.

the optical endoscope processing device 20 comprises a computer 84, a bus 85, and an external I/F 86. The computer 84 comprises a processor 88, a memory 90, and a storage 92. The processor 88, the memory 90, the storage 92, and the external I/F 86 are connected to a bus 85. The processor 88 controls the entire optical endoscope processing device 20. The memory 90 and the storage 92 are used by the processor 88.

The external I/F 86 controls the transmission and the reception of various types of information between one or more devices (hereinafter, also referred to as a "second external device") that are present outside the optical endoscope processing device 20 and the processor 88.

The reception device 64 is connected to the external I/F 86, as one of the second external devices, and the processor 88 acquires the instruction received by the reception device 64 via the external I/F 86 and executes processing in accordance with the acquired instruction.

A sensor 83 is connected to the external I/F 86, as one of the second external devices. The sensor 83 is provided in the image processing device 17, to detect whether the ultrasound endoscope 16 is connected to the image processing device 17 or the optical endoscope 300 is connected to the image processing device 17. Examples of a type of the sensor 83 include a microswitch, a proximity sensor, a magnetic sensor, and a photo sensor. The processor 88 acquires a detection result of the sensor 83 via the external I/F 86, and understands whether the ultrasound endoscope 16 is connected to the image processing device 17 or the optical endoscope 300 is connected to the image processing device 17, based on the acquired detection result.

In a case in which the ultrasound endoscope 16 is connected to the optical endoscope processing device 20, the camera 60 is connected to the external I/F 86, as one of the second external devices. The external I/F 86 controls the transmission and the reception of various types of information between the camera 60 and the processor 88. The processor 88 controls the camera 60 via the external I/F 86. In addition, the processor 88 acquires, via the external I/F 86, the electric signal obtained by imaging the inside of the upper gastrointestinal tract 28 via the camera 60, and generates the first optical image 29Abased on the acquired electric signal.

In a case in which the optical endoscope 300 is connected to the image processing device 17, the camera 352 is connected to the external I/F 86, as one of the second external devices. The external I/F 86 controls the transmission and the reception of various types of information between the camera 352 and the processor 88. The processor 88 controls the camera 352 via the external I/F 86. In addition, the processor 88 acquires, via the external I/F 86, the electric signal obtained by imaging the inside of the upper gastrointestinal tract 28 via the camera 352, and generates the second optical image 329A based on the acquired electric signal.

The light source device 22 is connected to the external I/F 86, as one of the second external devices, and the external I/F 86 controls the transmission and the reception of various types of information between the light source device 22 and the processor 88. In a case in which the ultrasound endoscope 16 is connected to the optical endoscope processing device 20, the light source device 22 supplies the light to the illumination device 58 under the control of the processor 88. In a case in which the optical endoscope 300 is connected to the optical endoscope processing device 20, the light source device 22 supplies the light to the illumination device 354 under the control of the processor 88. The illumination devices 58 and 354 emit the light supplied from the light source device 22.

The external I/F 70 is connected to the external I/F 86, as one of the second external devices, and the processor 88 transmits and receives various types of information to and from the processor 72 via the external I/Fs 70 and 88.

The reception device 64 is connected to the external I/F 86, as one of the second external devices, and the processor 88 acquires the instruction received by the reception device 64 via the external I/F 86 and executes processing in accordance with the acquired instruction.

The function expansion device 24 is connected to the external I/F 86, as one of the second external devices. As shown in Fig. 5 as an example, the function expansion device 24 comprises a computer 96 and an external I/F 98, and the external I/F 98 is connected to the external I/F 86.

The computer 96 comprises a processor 100, a memory 102, and a storage 104. The processor 100, the memory 102, the storage 104, and the external I/F 98 are connected to a bus 106. The processor 100 in the present embodiment is an example of a "processor" according to the present disclosure.

The external I/F 98 controls the transmission and the reception of various types of information between one or more devices (hereinafter also referred to as "third external devices") that are present outside the function expansion device 24 and the processor 100.

The external I/F 86 controls the transmission and the reception of various types of information between the processor 100 of the function expansion device 24 and the processor 88 (see Fig. 4) of the optical endoscope processing device 20 as one of the third external devices. The processor 100 acquires, for example, the medical image 29 (see Fig. 1) from the processor 88 of the optical endoscope processing device 20 via the external I/Fs 86 and 98. The processor 100 executes the processing using the medical image 29.

The display device 18 is connected to the external I/F 98, as one of the third external devices. The processor 100 displays various types of information (for example, medical image 29) on the display device 18 by controlling the display device 18 via the external I/F 98. The processor 100 displays, for example, the medical image 29 on the screen 35 of the display device 18. The example shown in Fig. 5 shows the ultrasound image 29B displayed in the first display region 35A, and the first optical image 29A displayed in the second display region 35B.

In recent years, the image processing device 17 may be connected to the display device 18 via an AI-BOX, which is a box-type device equipped with an AI function. The AI-BOX acquires the first optical image 29A and the ultrasound image 29B from the image processing device 17, and outputs the first optical image 29A and the ultrasound image 29B to the display device 18. In addition, the AI-BOX performs the recognition processing on the ultrasound image 29B by using AI and outputs a recognition processing result to the display device 18. The AI-BOX displays the first optical image 29A and the ultrasound image 29B on the screen 35 in a designated layout, and displays the recognition processing result on the ultrasound image 29B in a superimposed manner as a bounding box or the like.

As the specifications of the AI-BOX in the related art, there are a specification assuming that the ultrasound endoscope 16 is used and a specification assuming that the optical endoscope 300 is used. In the former specification, in a case in which the optical endoscope 300 is connected to the image processing device 17, the AI-BOX cannot display the second optical image 329A input from the image processing device 17 on the screen 35 or cannot display the recognition processing result of the recognition processing performed by the AI on the second optical image 329A on the screen 35. On the other hand, in the latter specification, in a case in which the ultrasound endoscope 16 is connected to the image processing device 17, the AI-BOX cannot display the first optical image 29A and the ultrasound image 29B input from the image processing device 17 on the screen 35 or cannot display the recognition processing result of the recognition processing performed by the AI on the ultrasound image 29B on the screen 35. As described above, the AI-BOX in the related art cannot allow the user 12 to visually recognize the information suitable for the type of the endoscope 15 connected to the image processing device 17.

Therefore, in view of such circumstances, in the present embodiment, as shown in Fig. 5 as an example, the medical support process is performed by the processor 100 of a device in which the AI-BOX of the related art is improved, that is, the function expansion device 24. A medical support program 108, a first recognition model 110A, and a second recognition model 110B are stored in the storage 104. The processor 100 reads out the medical support program 108 from the storage 104 and executes the readout medical support program 108 on the memory 102 to execute the medical support process. The medical support process is implemented by the processor 100 operating as a controller 100A and a recognition unit 100B in accordance with the medical support program 108 executed on the memory 102. As will be described in more detail later, the first recognition model 110A and the second recognition model 110B are used by the recognition unit 100B.

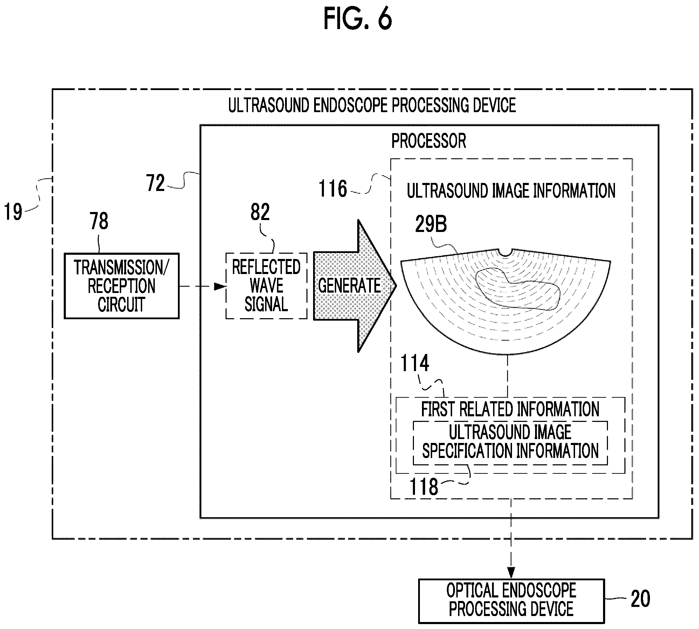

Fig. 6 shows an example of a processing content of the processor 72 in a case in which the processor 72 of the ultrasound endoscope processing device 19 acquires the reflected wave signal 82 from the transmission/reception circuit 78 in a state in which the ultrasound endoscope 16 is connected to the image processing device 17. As shown in Fig. 6, the processor 72 acquires the reflected wave signal 82 from the transmission/reception circuit 78, and generates the ultrasound image 29B based on the acquired reflected wave signal 82. The processor 72 generates ultrasound image information 116. The ultrasound image information 116 includes the ultrasound image 29B and first related information 114. The first related information 114 is information related to the ultrasound image 29B and is given to the ultrasound image 29B. Ultrasound image specification information 118 is included in the first related information 114. The ultrasound image specification information 118 is information for specifying that an image to which the first related information 114 is given is the ultrasound image 29B. The processor 72 outputs the ultrasound image information 116 to the optical endoscope processing device 20. The ultrasound image information 116 is generated at a first frame rate (for example, 15 frames/second) and output.

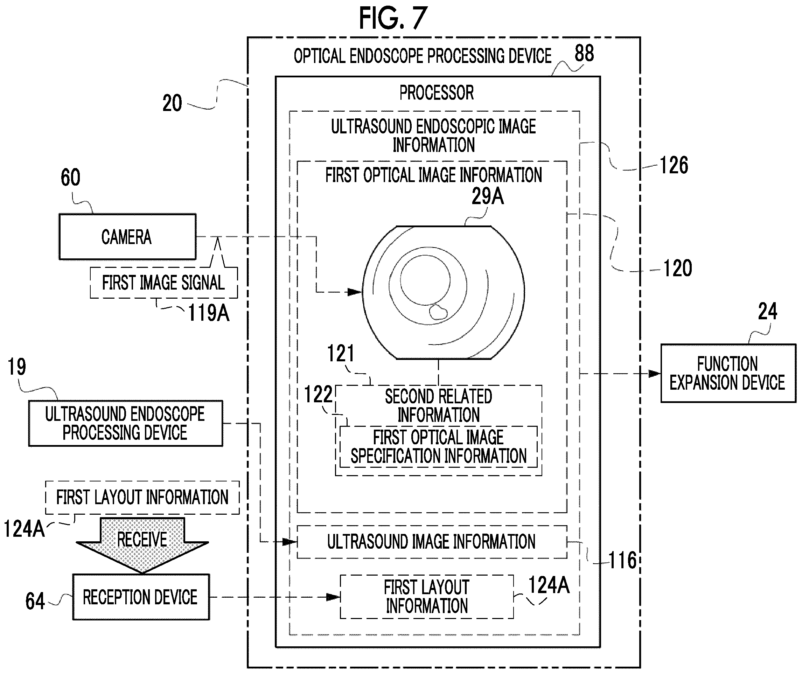

Fig. 7 shows an example of a processing content of the processor 88 of the optical endoscope processing device 20 in a state in which the ultrasound endoscope 16 is connected to the image processing device 17. As shown in Fig. 7, the processor 88 acquires a first image signal 119A, which is the electric signal obtained by imaging the inside of the upper gastrointestinal tract 28 via the camera 60, and generates the first optical image 29A based on the acquired first image signal 119A. In addition, the processor 88 generates first optical image information 120. The first optical image information 120 includes the first optical image 29A and second related information 121. The second related information 121 is information related to the first optical image 29A and is given to the first optical image 29A. First optical image specification information 122 is included in the second related information 121. The first optical image specification information 122 is information for specifying that an image to which the second related information 121 is given is the first optical image 29A. The first optical image information 120 is generated at a second frame rate (for example, 30 frames/second).

The processor 88 generates ultrasound endoscopic image information 126 and outputs the generated ultrasound endoscopic image information 126 to the function expansion device 24. The ultrasound endoscopic image information 126 is generated at the second frame rate and output.

The ultrasound endoscopic image information 126 includes the first optical image information 120 and first layout information 124A. The reception device 64 receives the first layout information 124A. The processor 88 acquires the first layout information 124A received by the reception device 64. The first layout information 124A is information indicating the layout of the first optical image 29A and the ultrasound image 29B in the screen 35. Examples of the first layout information 124A include information for an instruction to display the ultrasound image 29B in the first display region 35A, display the first optical image 29A in the second display region 35B, and display first reference information (here, as an example, information obtained by performing the recognition processing of the AI method on the ultrasound image 29B and expressing the recognition result in a text format), which is information to be referred to by the user 12 in performing the medical treatment, in the vicinity of the display location of the ultrasound image 29B in the first display region 35A. It should be noted that, here, although the form example is described in which the first layout information 124A is received by the reception device 64, this is merely an example, and the first layout information 124A may be determined in advance or may be changed in accordance with an operating state and/or a design change of the endoscope system 10.

The processor 88 acquires the ultrasound image information 116 from the ultrasound endoscope processing device 19. In a case in which a timing at which the ultrasound image information 116 is acquired and a timing at which the first optical image information 120 is generated match each other, the processor 88 includes the ultrasound image information 116 in the ultrasound endoscopic image information 126. The reason is that the frame rate (that is, the first frame rate) at which the ultrasound endoscopic image information 126 is generated is lower than the frame rate (that is, the second frame rate) at which the first optical image information 120 is generated.

The first image signal 119A in the present embodiment is an example of a "first image signal" according to the present disclosure. The ultrasound endoscopic image information 126 in the present embodiment is an example of "first image information" according to the present disclosure. In addition, the ultrasound image specification information 118 and the first optical image specification information 122 in the present embodiment are examples of "second specification information" according to the present disclosure. In addition, the first optical image 29A included in the first optical image information 120 of the ultrasound endoscopic image information 126 and the ultrasound image 29B included in the ultrasound image information 116 of the ultrasound endoscopic image information 126 in the present embodiment are examples of a "type of an image included in the first image information" according to the present disclosure.

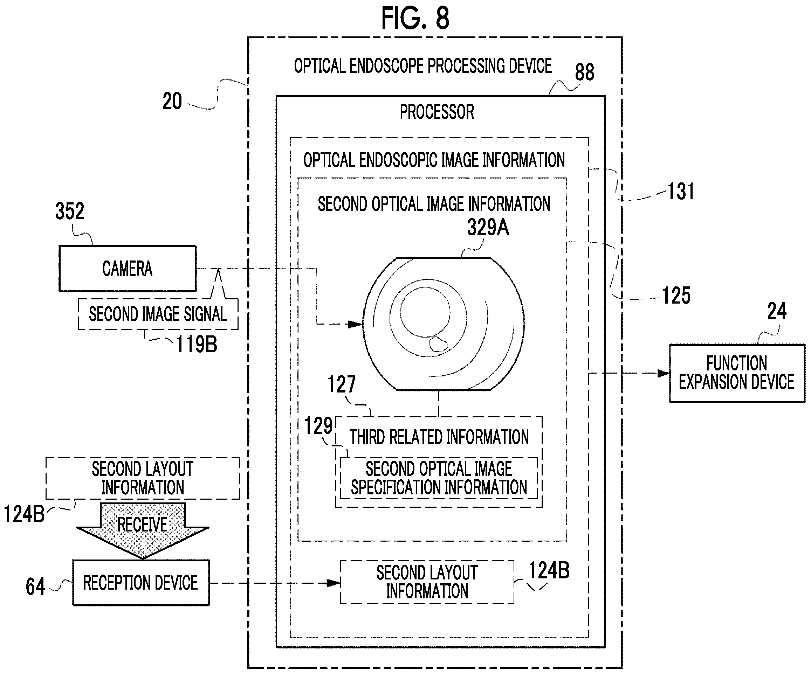

Fig. 8 shows an example of a processing content of the processor 88 of the optical endoscope processing device 20 in a state in which the optical endoscope 300 is connected to the optical endoscope processing device 20. As shown in Fig. 8, the processor 88 acquires a second image signal 119B, which is the electric signal obtained by imaging the inside of the upper gastrointestinal tract 28 via the camera 352, and generates the second optical image 329A based on the acquired second image signal 119B. In addition, the processor 88 generates second optical image information 125. The second optical image information 125 includes the second optical image 329A and third related information 127. The third related information 127 is information related to the second optical image 329A and is given to the second optical image 329A. Second optical image specification information 129 is included in the third related information 127. The second optical image specification information 129 is information for specifying that an image to which the third related information 127 is given is the second optical image 329A. The second optical image information 125 is generated at a third frame rate (for example, 30 frames/second).

The processor 88 generates optical endoscopic image information 131 and outputs the generated optical endoscopic image information 131 to the function expansion device 24. The optical endoscopic image information 131 is generated at the third frame rate and output.

The optical endoscopic image information 131 includes the second optical image information 125 and second layout information 124B. The reception device 64 receives the second layout information 124B. The processor 88 acquires the second layout information 124B received by the reception device 64. The second layout information 124B is information indicating the layout of the second optical image 329A in the screen 35. Examples of the second layout information 124B include information for an instruction to display the second optical image 329A in the first display region 35A and display second reference information (here, as an example, information in which a recognition result obtained by performing the recognition processing of the AI method on the second optical image 329A is expressed in a text format), which is information to be referred to by the user 12 in performing the medical treatment, in the second display region 35B. It should be noted that, here, although the form example is described in which the second layout information 124B is received by the reception device 64, this is merely an example, and the second layout information 124B may be determined in advance or may be changed in accordance with an operating state and/or a design change of the endoscope system 10.

The second image signal 119B in the present embodiment is an example of a "second image signal" according to the present disclosure. The optical endoscopic image information 131 in the present embodiment is an example of "second image information" according to the present disclosure. In addition, the second optical image specification information 129 in the present embodiment is an example of "second specification information" according to the present disclosure. In addition, the second optical image 329A included in the second optical image information 125 of the optical endoscopic image information 131 in the present embodiment is an example of a "type of an image included in the second image information" according to the present disclosure.

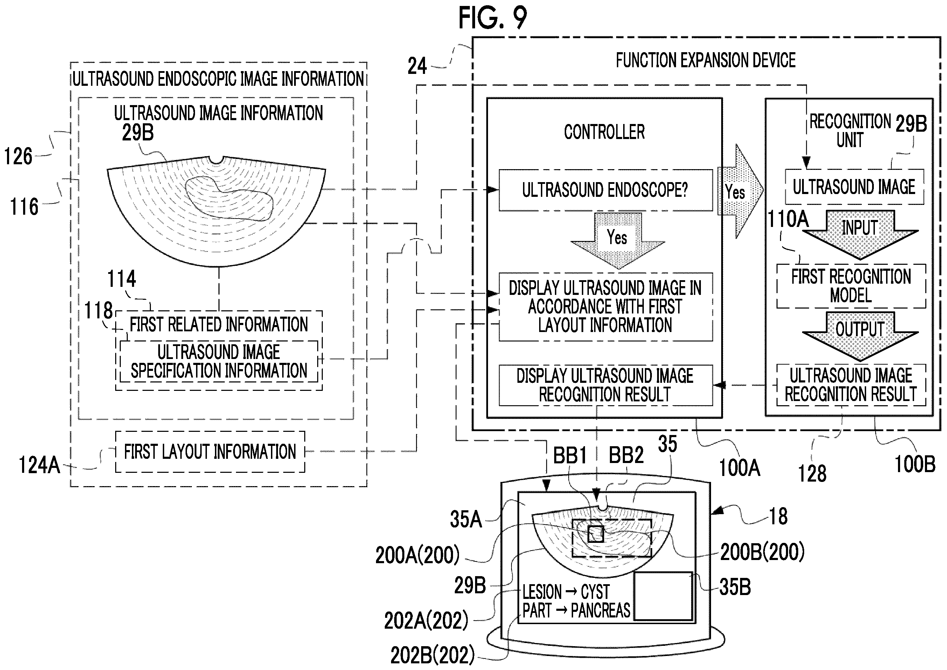

Fig. 9 shows an example of a processing content in a case in which the processing using the ultrasound image information 116 and the first layout information 124A, which are included in the ultrasound endoscopic image information 126, is performed by the function expansion device 24. As shown in Fig. 9, the controller 100A acquires the ultrasound endoscopic image information 126 from the optical endoscope processing device 20 (see Fig. 7) via the cable C2 (see Fig. 3), and acquires the ultrasound image specification information 118 from the first related information 114 included in the ultrasound image information 116 of the acquired ultrasound endoscopic image information 126. The controller 100A specifies that the ultrasound endoscope 16 is connected to the image processing device 17 based on the ultrasound image specification information 118.

Further, the controller 100A outputs the ultrasound image 29B included in the ultrasound image information 116 of the ultrasound endoscopic image information 126 to the display device 18. In the example shown in Fig. 9, the controller 100A acquires the first layout information 124Aincluded in the ultrasound endoscopic image information 126, and displays the ultrasound image 29B included in the ultrasound image information 116 of the ultrasound endoscopic image information 126 on the screen 35 in accordance with the acquired first layout information 124A. In the present embodiment, since the instruction to display the ultrasound image 29B in the first display region 35A is issued by the first layout information 124A, the controller 100A displays the ultrasound image 29B included in the ultrasound image information 116 of the ultrasound endoscopic image information 126 in the first display region 35A. The first display region 35A in the present embodiment is an example of a "first display region" according to the present disclosure.

The recognition unit 100B acquires the ultrasound image 29B included in the ultrasound image information 116 of the ultrasound endoscopic image information 126, and recognizes a feature region 200 shown in the ultrasound image 29B based on the ultrasound image 29B. Examples of the feature region 200 include a lesion 200A (for example, a cyst) and an inside a part 200B (for example, a pancreas) inside the body as a target of the examination via the ultrasound endoscope 16.

In order to implement the recognition of the feature region 200 based on the ultrasound image 29B, the recognition unit 100B recognizes the lesion 200A and the part 200B by using the AI method. The recognition processing using the first recognition model 110A is performed here.

The first recognition model 110A is a trained model for object recognition via a bounding box method using AI. The first recognition model 110A has been optimized by training a neural network through machine learning using first training data that is a data set including a plurality of data (that is, data for a plurality of frames) in which first example data and first correct answer data are associated with each other. That is, the first recognition model 110A is a trained model that has been optimized to receive input of the first example data to generate the first correct answer data.

The first example data is an image corresponding to the ultrasound image 29B (in other words, a sample image assuming the ultrasound image 29B). A first example of the image corresponding to the ultrasound image 29B is an ultrasound image that is actually obtained by an ultrasound upper endoscope. A second example of the image corresponding to the ultrasound image 29B is a virtually generated image (for example, an image generated by generative AI).

The first correct answer data refers to correct answer data (that is, an annotation) for the first example data. That is, the first correct answer data is information for specifying the lesion and the part inside the body, which are shown in the image used as the first example data, in a distinguished manner. Here, as an example of the first correct answer data, an annotation for specifying geometrical characteristics (for example, a position, a size, and a shape) of the lesion shown in the image used as the first example data, medical features of the lesion (for example, a type of the lesion and a subtype of the lesion), and the part inside the body is used.

The recognition unit 100B inputs the ultrasound image 29B to the first recognition model 110A. As a result, the first recognition model 110A recognizes the lesion 200A and the part 200B, which are shown in the input ultrasound image 29B, each time the ultrasound image 29B is input, generates an ultrasound image recognition result 128 that is the recognition result, and outputs the ultrasound image recognition result 128 to the display device 18.

The ultrasound image recognition result 128 includes bounding boxes BB1 and BB2 having different display aspects. The ultrasound image 29B is displayed in the first display region 35A of the display device 18, and, in this state, the bounding boxes BB1 and BB2 are displayed in a superimposed manner on the ultrasound image 29B. The bounding box BB 1 is a frame for specifying a position of the lesion 200A in the ultrasound image 29B, and the bounding box BB2 is a frame for specifying a position of the part 200B in the ultrasound image 29B. In the example shown in Fig. 9, a solid line indicates the bounding box BB1, and a broken line indicates the bounding box BB2. This display aspect is merely an example, and the bounding boxes BB1 and BB2 may be displayed in different colors or brightness, or only one of the bounding box BB 1 or the bounding box BB2 may be displayed in a blinking manner, and there are various ways to make the display aspects of the bounding box BB 1 and the bounding box BB2 different.

In addition, the ultrasound image recognition result 128 includes text 202. The text 202 includes first text 202A and second text 202B. The first text 202A is text for specifying the medical features of the lesion 200A. The second text 202B is text indicating a name of the part 200B.

The controller 100A displays the text 202 on the screen 35 in accordance with the first layout information 124A included in the ultrasound endoscopic image information 126. In the present embodiment, since the instruction to display the text 202, as the first reference information, around the display location of the ultrasound image 29B in the first display region 35A is issued by the first layout information 124A, the controller 100A displays the text 202 around the display location of the ultrasound image 29B in the first display region 35A. Therefore, the first text 202A and the second text 202B are displayed around the ultrasound image 29B in the first display region 35A.

The feature region 200 in the present embodiment is an example of a "second feature region" according to the present disclosure. In addition, the first recognition model 110A in the present embodiment is an example of a "trained model for an ultrasound image (ultrasound-image-trained model)" according to the present disclosure. In addition, the ultrasound image recognition result 128 in the present embodiment is an example of a "second recognition result" according to the present disclosure. In addition, the bounding boxes BB1 and BB2 in the present embodiment are examples of "second positional information" according to the present disclosure. In addition, the first text 202A in the present embodiment is an example of "second feature information" according to the present disclosure. In addition, the bounding box BB2 and the second text 202B in the present embodiment are examples of "second part information" according to the present disclosure.

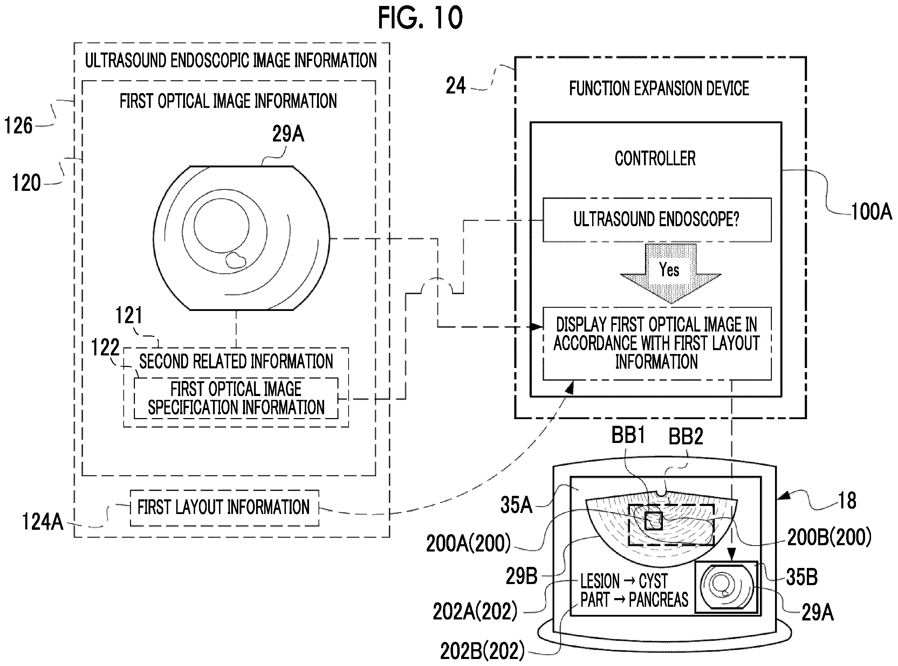

Fig. 10 shows an example of a processing content in a case in which the processing using the first optical image information 120 and the first layout information 124A, which are included in the ultrasound endoscopic image information 126, is performed by the function expansion device 24. As shown in Fig. 10, the controller 100A acquires the ultrasound endoscopic image information 126 from the optical endoscope processing device 20 (see Fig. 7) via the cable C2 (see Fig. 2), and acquires the first optical image specification information 122 from the second related information 121 included in the first optical image information 120 of the acquired ultrasound endoscopic image information 126. The controller 100A specifies that the ultrasound endoscope 16 is connected to the image processing device 17 based on the first optical image specification information 122.

Further, the controller 100A outputs the first optical image 29A included in the first optical image information 120 of the ultrasound endoscopic image information 126 to the display device 18. In the example shown in Fig. 10, the controller 100A acquires the first layout information 124A included in the ultrasound endoscopic image information 126, and displays the first optical image 29A included in the first optical image information 120 of the ultrasound endoscopic image information 126 on the screen 35 in accordance with the acquired first layout information 124A. In the present embodiment, since the instruction to display the first optical image 29A in the second display region 35B is issued by the first layout information 124A, the controller 100A displays the first optical image 29A included in the first optical image information 120 of the ultrasound endoscopic image information 126 in the second display region 35B.

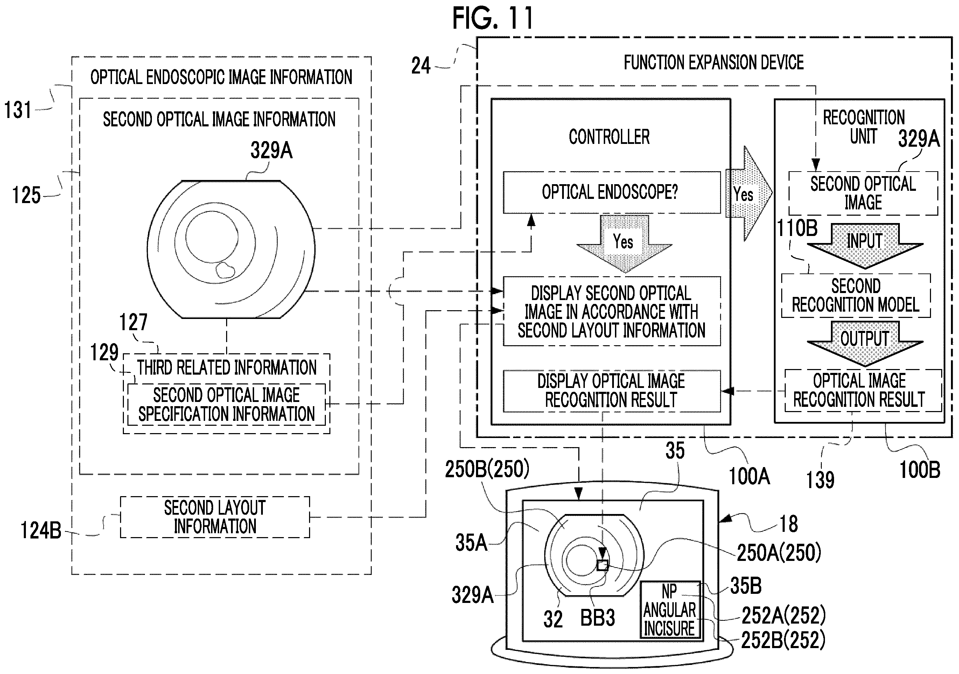

Fig. 11 shows an example of a processing content in a case in which the processing using the second optical image information 125 and the second layout information 124B, which are included in the optical endoscopic image information 131, is performed by the function expansion device 24. As shown in Fig. 11, the controller 100A acquires the optical endoscopic image information 131 from the optical endoscope processing device 20 (see Fig. 8) via the cable C2 (see Fig. 2), and acquires the second optical image specification information 129 from the third related information 127 included in the second optical image information 125 of the acquired optical endoscopic image information 131. The controller 100A specifies that the optical endoscope 300 is connected to the image processing device 17 based on the second optical image specification information 129.

Further, the controller 100A outputs the second optical image 329A included in the second optical image information 125 of the optical endoscopic image information 131 to the display device 18. In the example shown in Fig. 11, the controller 100A acquires the second layout information 124B included in the optical endoscopic image information 131, and displays the second optical image 329A included in the second optical image information 125 of the optical endoscopic image information 131 on the screen 35 in accordance with the acquired second layout information 124B. In the present embodiment, since the instruction to display the second optical image 329A in the first display region 35A is issued by the second layout information 124B, the controller 100A displays the second optical image 329A included in the second optical image information 125 of the optical endoscopic image information 131 in the first display region 35A.

Further, the recognition unit 100B acquires the second optical image 329A included in the second optical image information 125 of the optical endoscopic image information 131, and recognizes a feature region 250 shown in the second optical image 329A based on the second optical image 329A. Examples of the feature region 250 include a lesion 250A (for example, cancer) and a part 250B (for example, a part of the stomach) inside the body as a target of the examination via the optical endoscope 300.

In order to implement the recognition of the feature region 250 based on the second optical image 329A, the recognition unit 100B recognizes the lesion 250A and the part 250B by using the AI method. Here, the recognition processing using the second recognition model 110B is performed.

The second recognition model 110B is a trained model for object recognition using a bounding box method via AI, and is obtained by performing machine learning for the second optical image 329A. The second recognition model 110B has been optimized by training a neural network through machine learning using second training data that is a data set including a plurality of data (that is, data for a plurality of frames) in which second example data and second correct answer data are associated with each other. That is, the second recognition model 110B is a trained model that has been optimized to receive input of the second example data to generate the second correct answer data.

The second example data is an image corresponding to the second optical image 329A (in other words, a sample image assuming the second optical image 329A). A first example of the image corresponding to the second optical image 329A is an optical image that is actually obtained by an optical upper endoscope (for example, an optical endoscope having the same configuration as the optical endoscope 300). A second example of the image corresponding to the second optical image 329A is a virtually generated image (for example, an image generated by generative AI).

The second correct answer data refers to correct answer data (that is, an annotation) for the second example data. That is, the second correct answer data is information for specifying the lesion and the part inside the body, which are shown in the image used as the second example data, in a distinguished manner. Here, as an example of the second correct answer data, an annotation for specifying geometrical characteristics (for example, a position, a size, and a shape) of the lesion shown in the image used as the second example data, medical features of the lesion (for example, a type of the lesion and a subtype of the lesion), and the part inside the body is used.

The recognition unit 100B inputs the second optical image 329A to the second recognition model 110B. As a result, the second recognition model 110B recognizes the lesion 250A and the part 250B, which are included in the input second optical image 329A, each time the second optical image 329A is input, generates the optical image recognition result 139 that is the recognition result, and outputs the optical image recognition result 139 to the display device 18.

A bounding box BB3 is included in the optical image recognition result 139. The second optical image 329A is displayed in the first display region 35Aofthe display device 18, and, in this state, the bounding box BB3 is displayed in a superimposed manner on the second optical image 329A. The bounding box BB3 is a frame for specifying a position of the lesion 250A in the second optical image 329A.

In addition, the optical image recognition result 139 includes text 252. The text 252 includes third text 252A and fourth text 252B. The third text 252A is text for specifying medical features of the lesion 250A. The fourth text 252B is text indicating a name of the part 250B.

The controller 100A displays the text 252 on the screen 35 in accordance with the second layout information 124B included in the optical endoscopic image information 131. In the present embodiment, since the instruction to display the text 252 as the second reference information in the second display region 35B is issued by the second layout information 124B, the controller 100A displays the text 252 in the second display region 35B. Therefore, the third text 252A and the fourth text 252B are displayed in the second display region 35B.

The feature region 250 in the present embodiment is an example of a "first feature region" according to the present disclosure. In addition, the second recognition model 110B in the present embodiment is an example of a "trained model for an optical image (optical-image-trained model)" according to the present disclosure. In addition, the optical image recognition result 139 in the present embodiment is an example of a "first recognition result" according to the present disclosure. In addition, the bounding box BB3 in the present embodiment is an example of "first positional information" according to the present disclosure. In addition, the third text 252A in the present embodiment is an example of "first feature information" according to the present disclosure. In addition, the fourth text 252B in the present embodiment is an example of "first part information" according to the present disclosure.

In the example shown in Fig. 11, the form example is described in which the text 252 is displayed in the second display region 35B, but the text 252 may be displayed in a region (for example, the first display region 35A) other than the second display region 35B. In this case, the second layout information 124B need only include information for an instruction to display the text 252 in a region (for example, the first display region 35A) other than the second display region 35B. As described above, the controller 100A displays the text 252 at the position (for example, the first display region 35A) designated by the second layout information 124B.

In the present embodiment, as described above, in a case in which the optical endoscope 300 is connected to the image processing device 17, the optical image recognition result 139, which is obtained from the second recognition model 110B by inputting the second optical image 329A to the second recognition model 110B, is output to the display device 18. On the other hand, in a case in which the ultrasound endoscope 16 is connected to the image processing device 17, the first optical image 29A included in the ultrasound endoscopic image information 126 is not input to the second recognition model 110B. However, the form example in which the first optical image 29A is not input to the second recognition model 110B is merely an example, and in a case in which the ultrasound endoscope 16 is connected to the image processing device 17 (in other words, in a case in which the ultrasound image 29B is displayed in the first display region 35A), the information, which is obtained from the second recognition model 110B by inputting the first optical image 29A included in the ultrasound endoscopic image information 126 to the second recognition model 110B, may not be output to the display device 18. Here, the concept of "information is not output to the display device 18" also includes the concept that the information is displayed on the screen 35 at a display level that is not visually perceptible by the user 12 and the like.

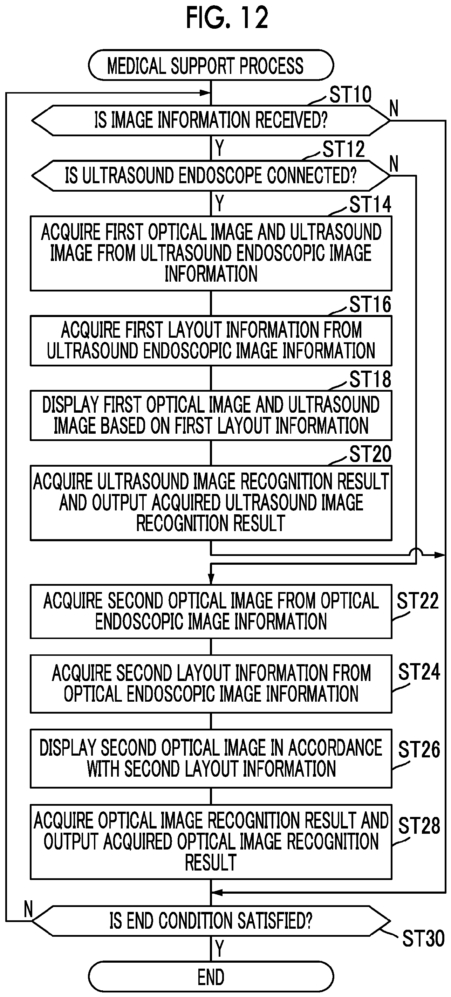

Next, an operation of a part of the endoscope system 10 according to the present disclosure will be described with reference to Fig. 12. A flow of the medical support process shown in Fig. 12 is an example of a "medical support method" according to the present disclosure.

In the medical support process shown in Fig. 12, first, in step ST10, the controller 100A determines whether the ultrasound endoscopic image information 126 or the optical endoscopic image information 131 is received by the external I/F 98 (see Fig. 5). In step ST10, in a case in which neither the ultrasound endoscopic image information 126 nor the optical endoscopic image information 131 is received by the external I/F 98, a No determination is made, and the medical support process proceeds to step ST30. In step ST10, in a case in which the ultrasound endoscopic image information 126 or the optical endoscopic image information 131 is received by the external I/F 98, a Yes determination is made, and the medical support process proceeds to step ST12.

In step ST12, the controller 100A determines whether the ultrasound endoscope 16 is connected to the image processing device 17. Here, in step ST10, in a case in which the ultrasound image specification information 118 is included in the ultrasound endoscopic image information 126 received by the external I/F 98, it is determined that the ultrasound endoscope 16 is connected to the image processing device 17. On the other hand, in step ST10, in a case in which the second optical image specification information 129 is included in the optical endoscopic image information 131 received by the external I/F 98, it is determined that the optical endoscope 300 is connected to the image processing device 17.

In step ST12, in a case in which the ultrasound endoscope 16 is not connected to the image processing device 17, that is, in a case in which the optical endoscope 300 is connected to the image processing device 17, a No determination is made, and the medical support process proceeds to step ST22. In step ST12, in a case in which the ultrasound endoscope 16 is connected to the image processing device 17, a Yes determination is made, and the medical support process proceeds to step ST14.

In step ST14, the controller 100A acquires the first optical image 29A and the ultrasound image 29B from the ultrasound endoscopic image information 126 received in step ST10 by the external I/F 98. After the processing in step ST14 is executed, the medical support process proceeds to step ST16.

In step ST16, the controller 100A acquires the first layout information 124A from the ultrasound endoscopic image information 126 received in step ST10 by the external I/F 98. After the processing in step ST16 is executed, the medical support process proceeds to step ST18.

In step ST18, the controller 100A displays the first optical image 29A and the ultrasound image 29B that are acquired in step ST14 on the screen 35 in accordance with the first layout information 124A acquired in step ST16. In the present embodiment, the ultrasound image 29B is displayed in the first display region 35A, and the first optical image 29A is displayed in the second display region 35B. After the processing in step ST18 is executed, the medical support process proceeds to step ST20.

In step ST20, the recognition unit 100B inputs the ultrasound image 29B to the first recognition model 110A to acquire the ultrasound image recognition result 128 from the first recognition model 110A. The controller 100A outputs the ultrasound image recognition result 128 acquired from the first recognition model 110A by the recognition unit 100B to the display device 18. As a result, the bounding box BB1, the bounding box BB2, the first text 202A, and the second text 202B are displayed as the ultrasound image recognition result 128 on the screen 35 (in the present embodiment, in the first display region 35A in the screen 35) (see Figs. 9 and 10). It should be noted that the first text 202A and the second text 202B are displayed around the display location of the ultrasound image 29B in the first display region 35A in accordance with the first layout information 124A.

In step ST22, the controller 100A acquires the second optical image 329A from the optical endoscopic image information 131 received in step ST10 by the external I/F 98. After the processing in step ST22 is executed, the medical support process proceeds to step ST24.

In step ST24, the controller 100A acquires the second layout information 124B from the optical endoscopic image information 131 received in step ST10 by the external I/F 98. After the processing in step ST24 is executed, the medical support process proceeds to step ST26.

In step ST26, the controller 100A displays the second optical image 329A acquired in step ST22 on the screen 35 in accordance with the second layout information 124B acquired in step ST24. In the present example, the second optical image 329A is displayed in the first display region 35A (see Fig. 11). After the processing in step ST26 is executed, the medical support process proceeds to step ST28.

In step ST28, the recognition unit 100B inputs the second optical image 329A to the second recognition model 110B to acquire the optical image recognition result 139 from the second recognition model 110B. Then, the controller 100A outputs the optical image recognition result 139, which is acquired from the second recognition model 110B by the recognition unit 100B, to the display device 18. As a result, the bounding box BB3, the third text 252A, and the fourth text 252B are displayed as the optical image recognition result 139 on the screen 35 (see Fig. 11). It should be noted that the third text 252A and the fourth text 252B are displayed in the second display region 35B in accordance with the second layout information 124B. After the processing in step ST28 is executed, the medical support process proceeds to step ST30.

In step ST30, the controller 100A determines whether a medical support process end condition is satisfied. A first example of the medical support process end condition is a condition in which a predetermined time (for example, 10 seconds) has elapsed since the execution of the medical support process is started. A second example of the medical support process end condition is a condition in which an instruction to end the medical support process is received by the reception device 64. In a case in which the medical support process end condition is not satisfied in step ST30, a No determination is made, and the medical support process proceeds to step ST10. In a case in which the medical support process end condition is satisfied in step ST30, a Yes determination is made, and the medical support process ends.

As described above, in the present embodiment, in a case in which the ultrasound endoscope 16 is connected to the image processing device 17, the ultrasound endoscopic image information 126 including the first optical image 29A and the ultrasound image 29B generated by performing the imaging via the ultrasound endoscope 16 is acquired by the controller 100A. Then, in a case in which the ultrasound endoscope 16 is connected to the image processing device 17, the first optical image 29A and the ultrasound image 29B included in the ultrasound endoscopic image information 126 are output to the display device 18.

On the other hand, in a case in which the optical endoscope 300 is connected to the image processing device 17, the optical endoscopic image information 131 including the second optical image 329A generated by performing the imaging via the optical endoscope 300 is acquired by the controller 100A. Then, in a case in which the optical endoscope 300 is connected to the image processing device 17, the second optical image 329A included in the optical endoscopic image information 131 is output to the display device 18. In addition, the optical image recognition result 139, which is acquired from the second recognition model 110B by inputting the second optical image 329A to the second recognition model 110B, is also output to the display device 18.

In this manner, the user 12 can visually recognize the information suitable for the type of the endoscope 15 connected to the image processing device 17. For example, in a case in which the ultrasound endoscope 16 is connected to the image processing device 17, the ultrasound image 29B is displayed in the first display region 35A, and the first optical image 29A is displayed in the second display region 35B. Therefore, the user 12 can visually recognize the ultrasound image 29B, which is displayed in the first display region 35A, and the first optical image 29A, which is displayed in the second display region 35B, as the information suitable for the ultrasound endoscope 16 connected to the image processing device 17. In addition, in a case in which the optical endoscope 300 is connected to the image processing device 17, the second optical image 329A is displayed in the first display region 35A, and the optical image recognition result 139 is displayed on the screen 35. Therefore, the user 12 can visually recognize the second optical image 329A, which is displayed in the first display region 35A, and the optical image recognition result 139, which is displayed on the screen 35, as information suitable for the optical endoscope 300 connected to the image processing device 17.

In addition, in the present embodiment, the function expansion device 24 and the image processing device 17 are separate bodies. In addition, the image processing device 17 includes an ultrasound endoscope processing device 19 and an optical endoscope processing device 20, and the ultrasound endoscope processing device 19 and the optical endoscope processing device 20 are separate bodies. Even in a case in which the function expansion device 24 and the image processing device 17 are separate bodies, or even in a case in which the function expansion device 24, the ultrasound endoscope processing device 19, and the optical endoscope processing device 20 are separate bodies, the user 12 can visually recognize the information suitable for the type of the endoscope 15 connected to the image processing device 17.

In addition, in the present embodiment, the function expansion device 24 is connected to the image processing device 17 by using the cable C2. Accordingly, the user 12 can selectively connect the ultrasound endoscope 16 and the optical endoscope 300 to the function expansion device 24 only by selectively connecting the ultrasound endoscope 16 and the optical endoscope 300 to the image processing device 17 without directly selectively connecting the ultrasound endoscope 16 and the optical endoscope 300 to the function expansion device 24. In this way, in a case in which the ultrasound endoscope 16 is connected to the image processing device 17 by connecting the function expansion device 24 to the image processing device 17 by using the cable C2, the ultrasound endoscopic image information 126 is acquired from the image processing device 17 by the controller 100A of the function expansion device 24 via the cable C2. In addition, in a case in which the optical endoscope 300 is connected to the image processing device 17, the optical endoscopic image information 131 is acquired from the image processing device 17 by the controller 100A of the function expansion device 24 via the cable C2. Therefore, the controller 100A of the function expansion device 24 can acquire the ultrasound endoscopic image information 126 in a case in which the ultrasound endoscope 16 is connected to the image processing device 17, and can acquire the optical endoscopic image information 131 in a case in which the optical endoscope 300 is connected to the image processing device 17, without the user 12 performing the work of selectively directly connecting the ultrasound endoscope 16 and the optical endoscope 300 to the function expansion device 24.

In addition, in the present embodiment, the ultrasound endoscope processing device 19 generates the ultrasound image 29B based on the reflected wave of the ultrasound emitted from the ultrasound endoscope 16. In addition, the optical endoscope processing device 20 generates the first optical image 29A based on the first image signal 119A obtained by performing the optical imaging via the ultrasound endoscope 16, and generates the second optical image 329A based on the second image signal 119B obtained by performing the optical imaging via the optical endoscope 300. Therefore, the ultrasound image 29B, the first optical image 29A, and the second optical image 329A can be generated with higher accuracy than in a case in which a general-purpose processing device, which is capable of generating both the ultrasound image and the optical image, generates the first optical image 29A, the ultrasound image 29B, and the second optical image 329A.

In addition, in the present embodiment, the optical endoscope processing device 20 is shared between a case in which the user 12 uses the ultrasound endoscope 16 and a case in which the user 12 uses the optical endoscope 300. As a result, it is possible to contribute to the improvement of the usability.

Further, in the present embodiment, the ultrasound endoscopic image information 126 and the optical endoscopic image information 131 are generated by the optical endoscope processing device 20. The processor 100 of the function expansion device 24 acquires the ultrasound endoscopic image information 126 and the optical endoscopic image information 131 from the optical endoscope processing device 20. Therefore, even in a case in which the ultrasound endoscope 16 is connected to the image processing device 17, or even in a case in which the optical endoscope 300 is connected to the image processing device 17, the processor 100 of the function expansion device 24 can appropriately acquire the ultrasound endoscopic image information 126 and the optical endoscopic image information 131.

In addition, in the present embodiment, the controller 100A of the function expansion device 24 refers to the ultrasound image specification information 118 included in the ultrasound endoscopic image information 126 and the second optical image specification information 129 included in the optical endoscopic image information 131, to specify whether the endoscope 15 connected to the image processing device 17 is the ultrasound endoscope 16 or the optical endoscope 300. Therefore, the controller 100A of the function expansion device 24 can easily specify whether the endoscope 15 connected to the image processing device 17 is the ultrasound endoscope 16 or the optical endoscope 300, as compared with a case in which the information for specifying whether the endoscope 15 connected to the image processing device 17 is the ultrasound endoscope 16 or the optical endoscope 300 is manually input to the function expansion device 24.

In addition, in the present embodiment, in a case in which the ultrasound endoscope 16 is connected to the image processing device 17, the first optical image 29A included in the ultrasound endoscopic image information 126 is not input to the second recognition model 110B. Since the second recognition model 110B is a trained model obtained by performing machine learning for the second optical image 329A, the information obtained from the second recognition model 110B by inputting the first optical image 29A to the second recognition model 110B has lower reliability than the information obtained from the second recognition model 110B by inputting the second optical image 329A to the second recognition model 110B. Therefore, by not inputting the first optical image 29A to the second recognition model 110B, the information having low reliability can be prevented from being provided to the user 12.

In addition, in a case in which the ultrasound endoscope 16 is connected to the image processing device 17 (in other words, in a case in which the ultrasound image 29B is displayed in the first display region 35A), the information, which is obtained from the second recognition model 110B by inputting the first optical image 29A included in the ultrasound endoscopic image information 126 to the second recognition model 110B, may not be output to the display device 18. In this way, in a case in which the ultrasound endoscope 16 is connected to the image processing device 17 (in other words, in a case in which the ultrasound image 29B is displayed in the first display region 35A), the user 12 can be prevented from recognizing the information (that is, the information having low reliability) obtained from the second recognition model 110B by inputting the first optical image 29A included in the ultrasound endoscopic image information 126 to the second recognition model 110B.

Further, in the present embodiment, the bounding box BB1, the bounding box BB2, the first text 202A, and the second text 202B are displayed on the screen 35 as the ultrasound image recognition result 128 obtained from the first recognition model 110A by inputting the ultrasound image 29B to the first recognition model 110A. The bounding box BB1 is information for specifying the position of the lesion 200A in the ultrasound image 29B, the bounding box BB2 is information for specifying the position of the part 200B in the ultrasound image 29B, the first text 202A is information indicating the medical features of the lesion 200A, and the second text 202B is information indicating the name of the part 200B. In this manner, the user 12 can visually recognize the position of the lesion 200A, the position of the part 200B, the medical features of the lesion 200A, and the name of the part 200B in the ultrasound image 29B displayed in the first display region 35A.

Further, in the present embodiment, the bounding box BB3, the third text 252A, and the fourth text 252B are displayed on the screen 35 as the optical image recognition result 139 obtained from the second recognition model 110B by inputting the second optical image 329A to the second recognition model 110B. The bounding box BB3 is information for specifying the position of the lesion 205A in the second optical image 329A, the third text 252A is information for specifying the medical features of the lesion 250A, and the fourth text 252B is information indicating the name of the part 250B. In this manner, the user 12 can visually recognize the position of the lesion 250A, the position of the part 250B, the medical features of the lesion 250A, and the name of the part 250B in the second optical image 329A displayed in the first display region 35A.

### Other Modification Examples

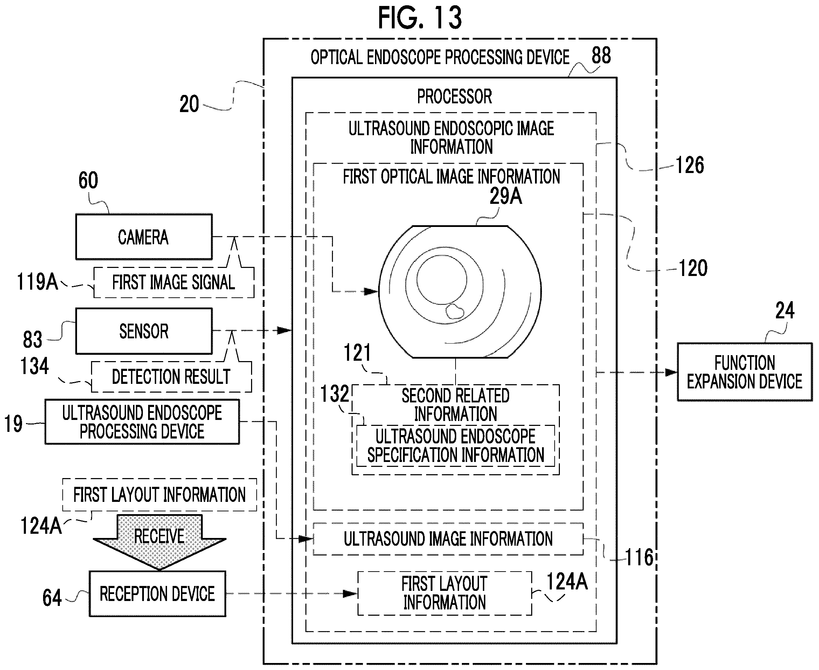

In the above-described embodiment, the form example is described in which the controller 100A of the function expansion device 24 specifies that the ultrasound endoscope 16 is connected to the image processing device 17 from the first optical image specification information 122 included in the second related information 121, but the present disclosure is not limited to this. As shown in Fig. 13 as an example, the second related information 121 may include ultrasound endoscope specification information 132, and the controller 100A may specify that the ultrasound endoscope 16 is connected to the image processing device 17 from the ultrasound endoscope specification information 132. The ultrasound endoscope specification information 132 is information for specifying that the type of the endoscope 15 connected to the image processing device 17 is the ultrasound endoscope 16, is generated by the processor 88 based on a detection result 134 of the sensor 83, and is included in the second related information 121. It should be noted that the first related information 114 may also include the ultrasound endoscope specification information 132. In this case as well, the controller 100A need only specify that the ultrasound endoscope 16 is connected to the image processing device 17 from the ultrasound endoscope specification information 132 included in the first related information 114. The ultrasound endoscope specification information 132 in the example shown in Fig. 13 is an example of "first specification information" according to the present disclosure.

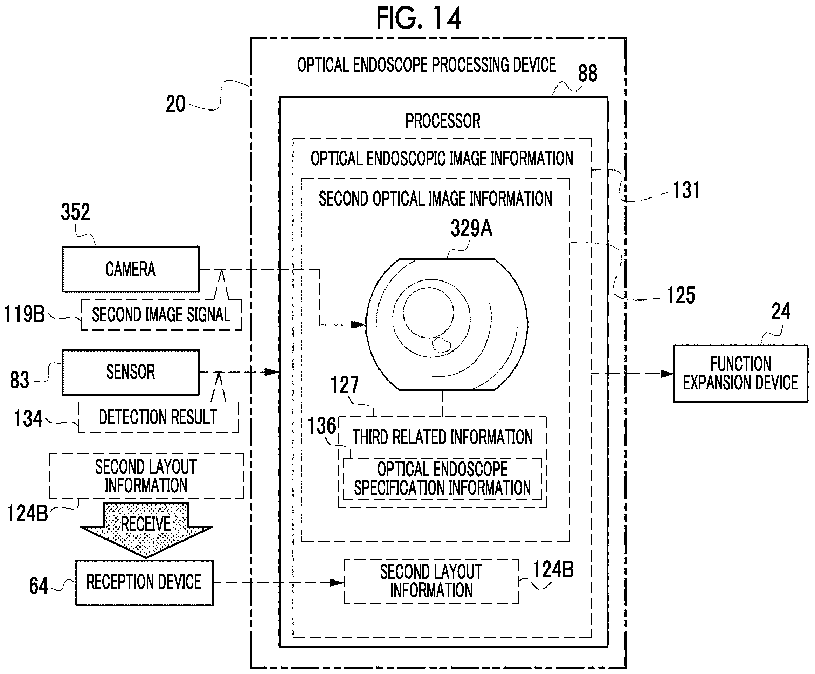

In the above-described embodiment, the form example is described in which the controller 100A of the function expansion device 24 specifies that the optical endoscope 300 is connected to the image processing device 17 from the second optical image specification information 129 included in the third related information 127, but the present disclosure is not limited to this. As shown in Fig. 14 as an example, the third related information 127 may include optical endoscope specification information 136, and the controller 100A may specify that the optical endoscope 300 is connected to the image processing device 17 from the optical endoscope specification information 136. The optical endoscope specification information 136 is information for specifying that the type of the endoscope 15 connected to the image processing device 17 is the optical endoscope 300, is generated by the processor 88 based on the detection result 134 by the sensor 83, and is included in the third related information 127. The optical endoscope specification information 136 in the example shown in Fig. 14 is an example of "first specification information" according to the present disclosure.

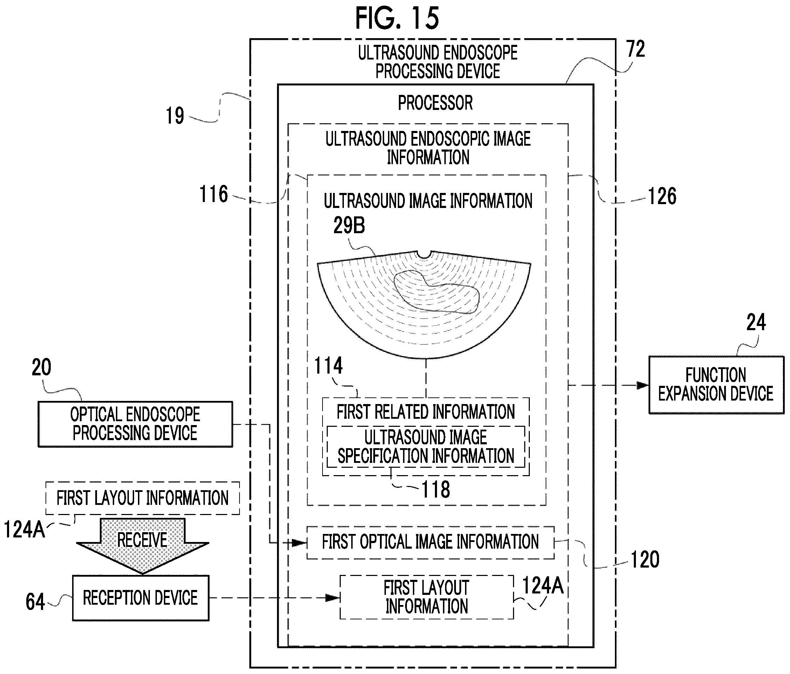

In the above-described embodiment, the form example is described in which the ultrasound image information 116 is input from the ultrasound endoscope processing device 19 to the optical endoscope processing device 20, and the processor 88 of the optical endoscope processing device 20 generates the ultrasound endoscopic image information 126 including the ultrasound image information 116, the first optical image information 120, and the first layout information 124A and outputs the generated ultrasound endoscopic image information 126 to the function expansion device 24, but the present disclosure is not limited to this. As shown in Fig. 15 as an example, the first optical image information 120 may be input from the optical endoscope processing device 20 to the ultrasound endoscope processing device 19, and the processor 72 of the ultrasound endoscope processing device 19 may generate the ultrasound endoscopic image information 126 including the ultrasound image information 116, the first optical image information 120, and the first layout information 124A and output the generated ultrasound endoscopic image information 126 to the function expansion device 24.

In addition, the second optical image information 125 may be input from the optical endoscope processing device 20 to the ultrasound endoscope processing device 19, and the processor 72 of the ultrasound endoscope processing device 19 may generate the optical endoscopic image information 131 including the second optical image information 125 and the second layout information 124B and output the generated optical endoscopic image information 131 to the function expansion device 24.

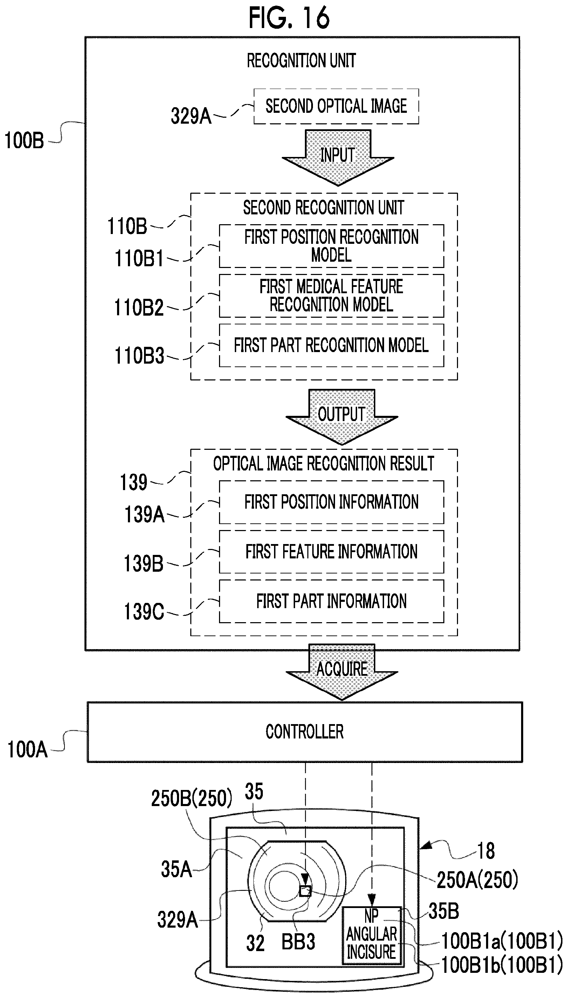

In the above-described embodiment, the form example is described in which the second recognition model 110B recognizes the feature region 250 shown in the second optical image 329A by inputting the second optical image 329A to the second recognition model 110B and outputs the optical image recognition result 139, but the present disclosure is not limited to this. As shown in Fig. 16 as an example, the second recognition model 110B may include a first position recognition model 110B1, a first medical feature recognition model 110B2, and a first part recognition model 110B3. The first position recognition model 110B 1 in the example shown in Fig. 16 is an example of a "first trained model" according to the present disclosure.

The first position recognition model 110B1 is a trained model that generates and outputs first positional information 139A, which is information for specifying the position of the feature region 250 in the second optical image 329A, in a case in which the second optical image 329A is input. The first medical feature recognition model 110B2 is a trained model that generates and outputs first feature information 139B, which is information for specifying the medical features of the feature region 250 included in the second optical image 329A, in a case in which the second optical image 329A is input. The first part recognition model 110B3 is a trained model that generates and outputs first part information 139C, which is information for specifying the part 250B shown in the second optical image 329A, in a case in which the second optical image 329A is input. The first positional information 139A in the example shown in Fig. 16 is an example of "first positional information" according to the present disclosure. In addition, the first feature information 139B in the example shown in Fig. 16 is an example of "first feature information" according to the present disclosure. In addition, the first part information 139C in the example shown in Fig. 16 is an example of "first part information" according to the present disclosure.

The optical image recognition result 139 includes the first positional information 139A, the first feature information 139B, and the first part information 139C. The controller 100A acquires the first positional information 139A, the first feature information 139B, and the first part information 139C, and outputs the first positional information 139A, the first feature information 139B, and the first part information 139C to the display device 18. As a result, the first positional information 139Ais displayed in the first display region 35A as the bounding box BB3, the first feature information 139B is displayed as the third text 252A in the second display region 35B, and the first part information 139C is displayed as the fourth text 252B in the second display region 35B.

As described above, in a case in which the first position recognition model 110B1, the first medical feature recognition model 110B2, and the first part recognition model 110B3 are used, the first positional information 139A, the first feature information 139B, and the first part information 139C can be generated with higher accuracy than in a case in which a single trained model generates the first positional information 139A, the first feature information 139B, and the first part information 139C.

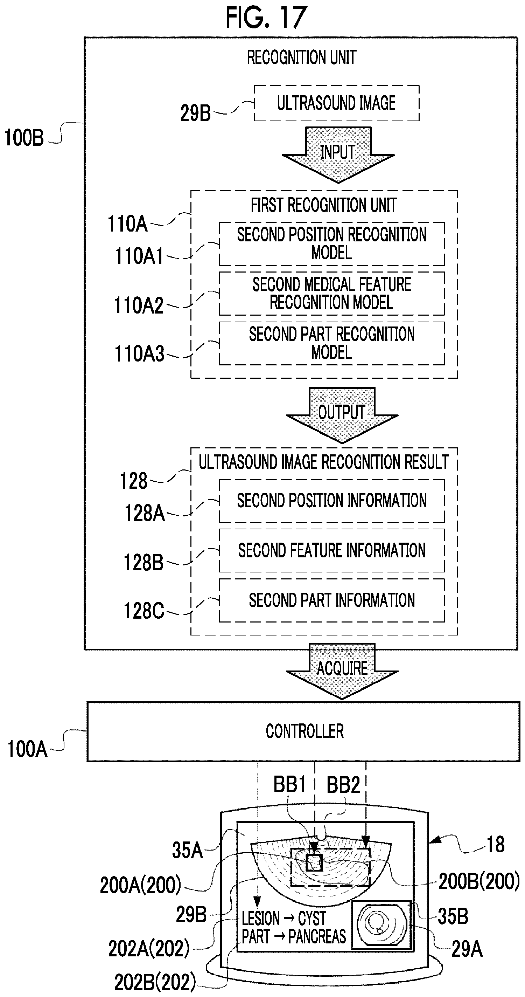

In the above-described embodiment, the form example is described in which the ultrasound image 29B is input to the first recognition model 110A and causes the first recognition model 110A to recognize the feature region 200 shown in the ultrasound image 29B and output the ultrasound image recognition result 128, but the present disclosure is not limited to this. As shown in Fig. 17 as an example, the first recognition model 110A may include a second position recognition model 110A1, a second medical feature recognition model 110A2, and a second part recognition model 110A3. The second position recognition model 110A1 in the example shown in Fig. 17 is an example of a "second trained model" according to the present disclosure. The second position recognition model 110A1 is a trained model that generates and outputs second positional information 128A, which is information for specifying the position of the feature region 200 in the ultrasound image 29B, in a case in which the ultrasound image 29B is input. The second medical feature recognition model 110A2 is a trained model that generates and outputs second feature information 128B, which is information for specifying a medical feature of the feature region 200 shown in the ultrasound image 29B, in a case in which the ultrasound image 29B is input. The second part recognition model 110A3 is a trained model that generates and outputs second part information 128C, which is information for specifying the part 200B shown in the ultrasound image 29B, in a case in which the ultrasound image 29B is input. The second positional information 128A in the example shown in Fig. 17 is an example of "second positional information" according to the present disclosure. In addition, the second feature information 128B in the example shown in Fig. 17 is an example of "second feature information" according to the present disclosure. In addition, the second part information 128C in the example shown in Fig. 17 is an example of "second part information" according to the present disclosure.

The ultrasound image recognition result 128 includes the second positional information 128A, the second feature information 128B, and the second part information 128C. The controller 100A acquires the second positional information 128A, the second feature information 128B, and the second part information 128C, and outputs the second positional information 128A, the second feature information 128B, and the second part information 128C to the display device 18. As a result, the second positional information 128A is displayed as the bounding boxes BB 1 and BB2 in the first display region 35A, the second feature information 128B is displayed as the first text 202A in the first display region 35A, and the second part information 128C is displayed as the second text 202B in the first display region 35A.

As described above, in a case in which the second position recognition model 110A1, the second medical feature recognition model 110A2, and the second part recognition model 110A3 are used, the second positional information 128A, the second feature information 128B, and the second part information 128C can be generated with higher accuracy than in a case in which a single trained model generates the second positional information 128A, the second feature information 128B, and the second part information 128C.

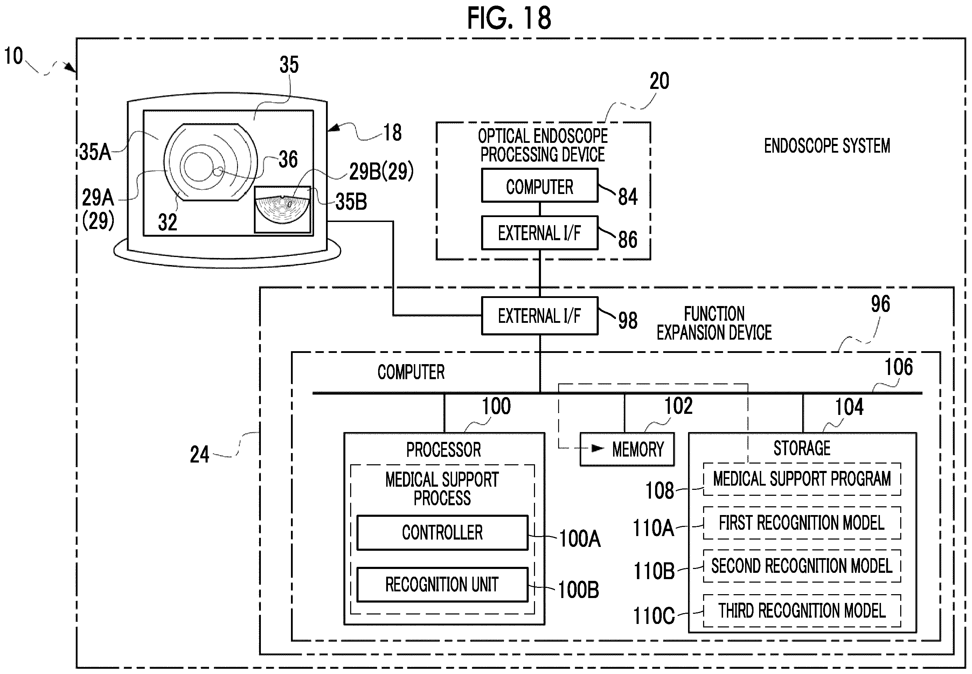

In the above-described embodiment, the form example is described in which the recognition processing using the first recognition model 110A is performed on the ultrasound image 29B, and the recognition processing using the second recognition model 110B is performed on the second optical image 329A, but the present disclosure is not limited to this. For example, the recognition processing of the AI method may also be performed on the first optical image 29A. In this case, as shown in Fig. 18 as an example, the third recognition model 110C is stored in the storage 104, and the recognition processing using the third recognition model 110C is performed on the first optical image 29A. The second recognition model 110B is a trained model obtained by performing machine learning for the second optical image 329A, but the third recognition model 110C is a trained model obtained by performing machine learning for the first optical image 29A.

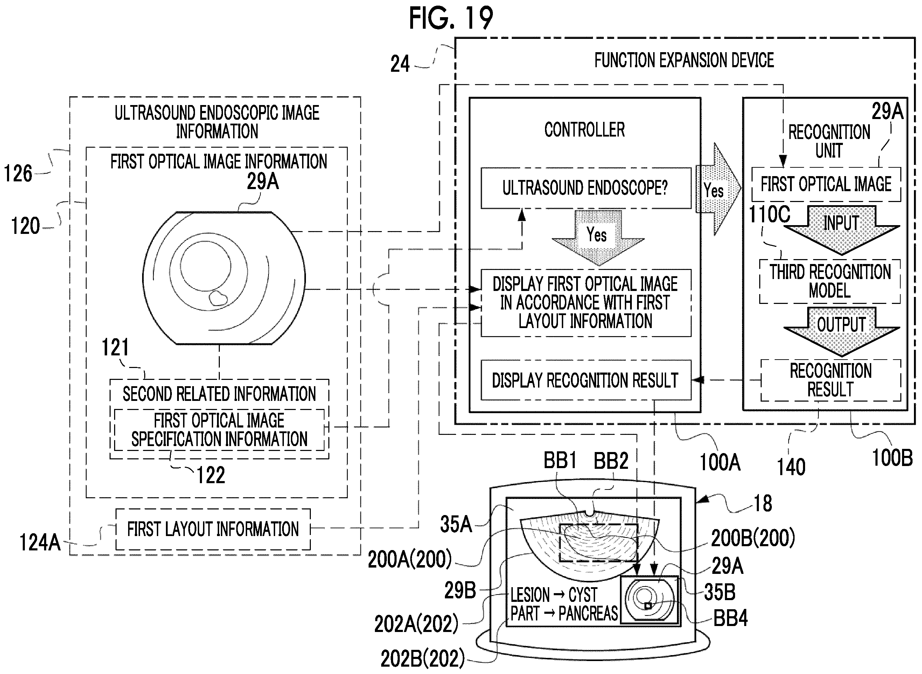

As shown in Fig. 19 as an example, in a case in which the controller 100A specifies that the ultrasound endoscope 16 is connected to the image processing device 17, the recognition unit 100B acquires the first optical image 29A included in the first optical image information 120 of the ultrasound endoscopic image information 126. Then, the recognition unit 100B inputs the first optical image 29A to the third recognition model 110C and causes the third recognition model 110C to generate the recognition result 140. The recognition result 140 is a result of recognizing the feature region (for example, the lesion) shown in the first optical image 29A. For example, the recognition result 140 includes information for specifying the position of the feature region in the first optical image 29A, information for specifying the medical features of the feature region shown in the first optical image 29A, and information for specifying the part (for example, an organ) shown in the first optical image 29A. The controller 100A outputs the recognition result 140 to the display device 18. As a result, for example, a bounding box BB4 is displayed in the second display region 35B as the information for specifying the position of the feature region in the first optical image 29A. In the example shown in Fig. 19, the bounding box BB4 is displayed in a superimposed manner on the first optical image 29A displayed in the second display region 35B.

In the above-described embodiment, the convex type ultrasound endoscope (for example, the ultrasound endoscope processing device 19) is described, but the present disclosure is established even in a case in which a radial type ultrasound endoscope (that is, an ultrasound endoscope comprising a radial type ultrasound probe) is used. In the radial type ultrasound endoscope (for example, the ultrasound endoscope in which the radial type ultrasound probe is provided on a base end side of an optical camera (in other words, an ultrasound endoscope in which an optical camera is provided on a distal end side of a cylindrical radial type ultrasound probe)), a case is also assumed in which the appearance of an optical image obtained by the optical camera provided in the radial type ultrasound endoscope is almost the same as the optical image (for example, the second optical image 329A) obtained by the optical endoscope (for example, the optical endoscope 300). In such a case, the second recognition model 110B used as a recognizer for the optical image obtained by the optical endoscope may be applied to the optical image obtained by the radial type ultrasound endoscope.

In the above-described embodiment, the form example is described in which the controller 100A of the function expansion device 24 specifies that the ultrasound endoscope 16 is connected to the image processing device 17 from the first optical image specification information 122 included in the second related information 121, or specifies that the optical endoscope 300 is connected to the image processing device 17 from the second optical image specification information 129 included in the third related information 127, but the present disclosure is not limited to this. For example, the image processing device 17 may be configured to specify whether the ultrasound endoscope 16 is connected to the image processing device 17 or the optical endoscope 300 is connected to the image processing device 17 by analyzing the image included in the ultrasound endoscopic image information 126 or the optical endoscopic image information 131.

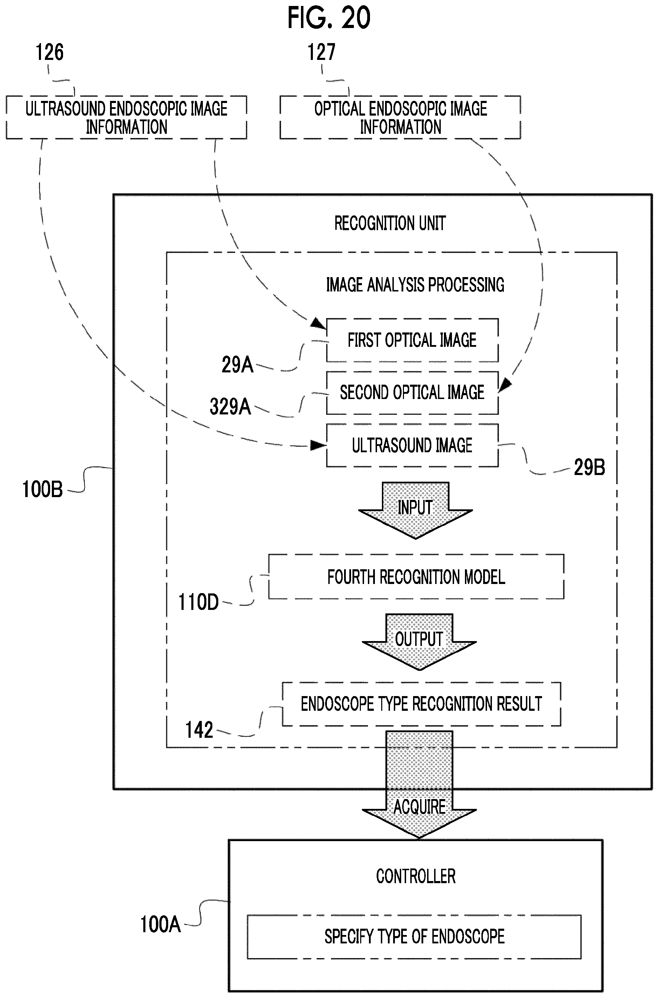

Fig. 20 shows a form example in which the type of the endoscope 15 connected to the image processing device 17 is specified by analysis using AI. In the example shown in Fig. 20, the recognition unit 100B uses a fourth recognition model 110D. The fourth recognition model 110D is a trained model that recognizes the type of the endoscope 15 used to obtain the input image, generates an endoscope type recognition result 142 that is a recognition result, and outputs the endoscope type recognition result 142.

The first optical image 29A, the second optical image 329A, or the ultrasound image 29B is input to the fourth recognition model 110D. For example, the fourth recognition model 110D recognizes that the type of the endoscope 15 connected to the image processing device 17 is the ultrasound endoscope 16 in a case in which the first optical image 29A or the ultrasound image 29B is input. In addition, the fourth recognition model 110D recognizes that the type of the endoscope 15 connected to the image processing device 17 is the optical endoscope 300 by inputting the second optical image 329A. The controller 100A acquires the endoscope type recognition result 142 and specifies the type of the endoscope 15 connected to the image processing device 17 from the acquired endoscope type recognition result 142.

In this way, the controller 100A of the function expansion device 24 can easily specify whether the endoscope 15 connected to the image processing device 17 is the ultrasound endoscope 16 or the optical endoscope 300, as compared with a case in which the information for specifying whether the endoscope 15 connected to the image processing device 17 is the ultrasound endoscope 16 or the optical endoscope 300 is manually input to the function expansion device 24.

It should be noted that, here, although the processing of recognizing the type of the endoscope 15 connected to the image processing device 17 by using the AI method is described, this is merely an example, and the present disclosure is established even in a case in which processing of recognizing the type of the endoscope 15 connected to the image processing device 17 by using a non-AI method (for example, processing that does not use AI, such as template matching) is used.

In the above-described embodiment, the trained model for object recognition using the bounding box method via AI is described, but the present disclosure is established by using a trained model for object recognition using a segmentation method via AI instead of the trained model for object recognition using the bounding box method via AI or together with the trained model for object recognition using the bounding box method via AI.

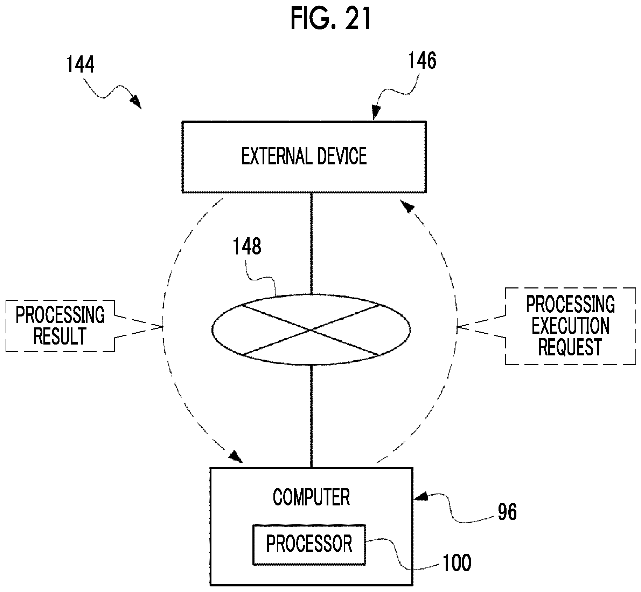

In the above-described embodiment, the form example is described in which the medical support process is executed by the computer 96, but the present disclosure is not limited thereto, and at least a part of the processing included in the medical support process may be executed by a device provided outside the computer 96. Hereinafter, an example of such a case will be described with reference to Fig. 21.

Fig. 21 is a conceptual diagram showing an example of a configuration of an endoscope system 144. The endoscope system 144 is an example of an "endoscope system" according to the present disclosure. The endoscope system 144 is different from the endoscope system 10 according to the above-described embodiment in that an external device 146 is provided.

The external device 146 is connected communicably to the computer 96 via a network 148 (for example, a WAN and/or a LAN).

Examples of the external device 146 include at least one server that directly or indirectly transmits and receives data to and from the computer 96 via the network 148. The external device 146 receives a processing execution instruction issued from the processor 100 of the computer 96 via the network 148. The external device 146 executes processing corresponding to the received processing execution instruction and transmits a processing result to the computer 96 via the network 148. In the computer 96, the processor 100 receives the processing result transmitted from the external device 146 via the network 148, and executes processing using the received processing result.

Examples of the processing execution instruction include an instruction to cause the external device 146 to execute at least a part of the medical support process. A first example of at least a part of the medical support process (that is, the processing to be executed by the external device 146) is the recognition processing using the first recognition model 110A, the second recognition model 110B, the third recognition model 110C, and/or the fourth recognition model 110D. In this case, the external device 146 executes the recognition processing in accordance with the processing execution instruction issued from the processor 100 via the network 148, and transmits the recognition result (for example, the ultrasound image recognition result 128, the optical image recognition result 139, the recognition result 140, and/or the endoscope type recognition result 142) to the computer 96 via the network 148. In the computer 96, the processor 100 receives the recognition result and executes the same processing as in the above-described embodiment by using the received recognition result.

A second example of at least a part of the medical support process (that is, the processing to be executed by the external device 146) is specification processing of specifying the type of the endoscope 15 connected to the image processing device 17. In this case, the external device 146 executes the specification processing in accordance with the processing execution instruction issued from the processor 100 via the network 148, and transmits the processing result (for example, the information for specifying the type of the endoscope 15 connected to the image processing device 17) to the computer 96 via the network 148. In the computer 96, the processor 100 receives the processing result and executes the same processing as in the above-described embodiment by using the received processing result.

The external device 146 is implemented by, for example, cloud computing. It should be noted that the cloud computing is merely an example, and the external device 146 may be implemented by network computing, such as fog computing, edge computing, or grid computing. At least one personal computer or the like may be used as the external device 146, instead of the server. Further, an operation device having a communication function equipped with a plurality of types of AI functions may be used.

In the above-described embodiment, the form example is described in which the medical support program 108 is stored in the storage 104, but the present disclosure is not limited thereto. For example, the medical support program 108 may be stored in a portable computer-readable non-transitory storage medium such as an SSD or a USB memory. The medical support program 108, which is stored in the non-transitory storage medium, is installed in the computer 96 of the endoscope system 10. The processor 100 executes the medical support process in accordance with the medical support program 108.

The medical support program 108 may be stored in a storage device of another computer or server device connected to the endoscope system 10 via a network, and the medical support program 108 may be downloaded in response to a request from the endoscope system 10 and installed in the computer 96.

It should be noted that it is not necessary to store the entire medical support program 108 in a storage device of another computer or server device connected to the endoscope system 10 or to store the entire medical support program 108 in the storage 104, and a part of the medical support program 108 may be stored.

Various processors described below can be used as the hardware resource for executing the medical support process. Examples of the processor include a CPU which is a general-purpose processor that functions as the hardware resource executing the medical support process by executing software, that is, a program. In addition, examples of the processor include a dedicated electric circuit which is a processor of which a circuit configuration is specially designed for executing specific processing, such as an FPGA, a PLD, or an ASIC. Any processor has a memory built in or connected to it, and any processor executes the medical support process by using the memory.

The hardware resource for executing the medical support process may be configured by one of the various processors or by combining two or more processors of the same type or different types (for example, by combining a plurality of FPGAs or by combining a CPU and an FPGA). The hardware resource for executing the medical support process may also be one processor.

A first example of the configuration using one processor is a form in which one processor is configured by combining one or more CPUs and software, and the processor functions as the hardware resource for executing the medical support process. As a second example, as typified by an SoC or the like, there is a form in which a processor that implements all functions of a system including a plurality of hardware resources executing the medical support process with one IC chip is used. As described above, the medical support process is implemented by using one or more of the various processors as the hardware resource.

As a hardware structure of these various processors, more specifically, an electric circuit in which circuit elements, such as semiconductor elements, are combined can be used. Further, the medical support process is merely an example. Therefore, it goes without saying that unnecessary steps may be deleted, new steps may be added, or the processing order may be changed within a range that does not deviate from the gist.

The above-described contents and the above-shown contents are the detailed description of the parts according to the present disclosure, and are merely examples of the present disclosure. For example, the description of the configuration, the function, the operation, and the effect is the description of examples of the configuration, the function, the operation, and the effect of the parts according to the present disclosure. Accordingly, it goes without saying that unnecessary parts may be deleted, new elements may be added, or replacements may be made with respect to the above-described contents and the above-shown contents within a range that does not deviate from the gist of the present disclosure. In addition, in order to avoid complications and facilitate understanding of the parts according to the present disclosure, the description of common technical knowledge or the like, which does not particularly require the description for enabling the implementation of the present disclosure, is omitted in the above-described contents and the above-shown contents.

All of the documents, the patent applications, and the technical standards described in the present specification are incorporated into the present specification by reference to the same extent as in a case in which the individual documents, patent applications, and technical standards are specifically and individually stated to be described by reference.

In regard to the above-described embodiment, the following supplementary notes will be further disclosed.

### Supplementary Note 1

A medical support device comprising: a processor, in which the processor is configured to: acquire a first optical image and an ultrasound image, which are generated by performing imaging via an ultrasound endoscope from an image processing device to which the ultrasound endoscope is connectable; output the first optical image and the ultrasound image to a display device; and output a recognition result, which is obtained from a trained model for an ultrasound image by inputting the ultrasound image to the trained model for an ultrasound image, to the display device in a case in which an instruction to display the ultrasound image in a first display region of the display device is issued.

### Supplementary Note 2

The medical support device according to supplementary note 1, in which the processor is configured to output a recognition result, which is obtained from a trained model for an optical image by inputting the first optical image to the trained model for an optical image, to the display device in a case in which an instruction to display the first optical image in the first display region is issued.

### Supplementary Note 3

The medical support device according to supplementary note 1 or 2, in which the processor is configured to not output a recognition result, which is obtained from a trained model for an optical image by inputting the first optical image to the trained model for an optical image, to the display device in a case in which the instruction to display the ultrasound image in the first display region is issued.

## Claims

1. A medical support device (24) comprising:
a processor (100),
wherein the processor (100) is configured to:
acquire first image information (126) including a first optical image (29A) and an ultrasound image (29B), which are generated by performing imaging via an ultrasound endoscope (16), in a case in which the ultrasound endoscope (16) is connected to an image processing device (17) to which the ultrasound endoscope (16) and an optical endoscope (300) are connectable;
acquire second image information (131) including a second optical image (329A), which is generated by performing imaging via the optical endoscope (300), in a case in which the optical endoscope (300) is connected to the image processing device (17);
output the first optical image (29A) and the ultrasound image (29B), which are included in the first image information (126), to a display device (18) in a case in which the ultrasound endoscope (16) is connected to the image processing device (17); and
output the second optical image (329A), which is included in the second image information (131), and a first recognition result (139), which is obtained by inputting the second optical image (329A) to an optical-image-trained model (110B) and causing the optical-image-trained model (110B) to recognize a first feature region (250) shown in the second optical image (329A), to the display device (18) in a case in which the optical endoscope (300) is connected to the image processing device (17).

2. The medical support device (24) according to claim 1,
wherein the medical support device (24) and the image processing device (17) are separate bodies.

3. The medical support device (24) according to claim 1,
wherein the medical support device (24) is connected to the image processing device (17) by using a cable (C1, C2)), and
the processor (100) is configured to acquire the first image information (126) and the second image information (131) from the image processing device (17) via the cable (C1, C2).

4. The medical support device (24) according to claim 1,
wherein the image processing device (17) includes a first image processing device (19) and a second image processing device (20),
the first image processing device (19) generates the ultrasound image (29B) based on a reflected wave of ultrasound emitted from the ultrasound endoscope (16), and
the second image processing device (20) generates the first optical image (29A) based on a first image signal (119A) obtained by performing optical imaging via the ultrasound endoscope (16) and generates the second optical image (329A) based on a second image signal (119B) obtained by performing optical imaging via the optical endoscope (300).

5. The medical support device (24) according to claim 4,
wherein the first image information (126) is generated by the first image processing device (19) or the second image processing device (20),
the second image information (131) is generated by the first image processing device (19) or the second image processing device (20), and
the processor (100) is configured to:
acquire the first image information (126) from the first image processing device (19) or the second image processing device (20); and
acquire the second image information (131) from the first image processing device (19) or the second image processing device (20).

6. The medical support device (24) according to claim 4,
wherein the first image processing device (19), the second image processing device (20), and the medical support device (24) are separate bodies.

7. The medical support device (24) according to claim 1,
wherein each of the first image information (126) and the second image information (131) includes first specification information (132) and/or second specification information (122),
the first specification information (132) is information for specifying a type of an endoscope connected to the image processing device (17),
the second specification information (122) is information for specifying a type of an image included in the first image information (126) or the second image information (131),
the types of the endoscope are the ultrasound endoscope (16) and the optical endoscope (300),
the types of the image are the ultrasound image (29B), the first optical image (29A), and the second optical image (329A), and
the processor (100) is configured to specify whether the ultrasound endoscope (16) is connected to the image processing device (17) or the optical endoscope (300) is connected to the image processing device (17), based on the first specification information (132) and/or the second specification information (122).

8. The medical support device (24) according to claim 1,
wherein the processor (100) is configured to specify whether the ultrasound endoscope (16) is connected to the image processing device (17) or the optical endoscope (300) is connected to the image processing device (17), by analyzing an image included in the first image information (126) or the second image information (131).

9. The medical support device (24) according to claim 1,
wherein the processor (100) is configured to not output information, which is obtained from the optical-image-trained model (110B) by inputting the first optical image (29A) included in the first image information (126) to the optical-image-trained model (110B), to the display device (18) in a case in which the ultrasound endoscope (16) is connected to the image processing device (17).

10. The medical support device (24) according to claim 1,
wherein the processor (100) is configured to not output information, which is obtained from the optical-image-trained model (110B) by inputting the first optical image (29A) included in the first image information (126) to the optical-image-trained model (110B), to the display device (18) in a case in which the ultrasound image (29B) is displayed in a first display region (35A) of the display device (18).

11. The medical support device (24) according to claim 9,
wherein the optical-image-trained model (110B) is a trained model obtained by performing machine learning for the second optical image (329A).

12. The medical support device (24) according to claim 1,
wherein the first recognition result (139) includes first positional information (BB3) for specifying a position of the first feature region (250), first feature information (252A) for specifying a medical feature of the first feature region (250), and/or first part information (252B) for specifying a part inside a body as a target of an examination via the optical endoscope (300).

13. The medical support device (24) according to claim 12,
wherein the optical-image-trained model (110B) includes a first trained model (110B 1) to which the second optical image (329A) is input, and
the first trained model (110B1) generates the first positional information (BB3) based on the input second optical image.

14. The medical support device (24) according to claim 1,
wherein the processor (100) is configured to output a second recognition result (128), which is obtained by inputting the ultrasound image (29B) included in the first image information (126) to an ultrasound-image-trained model (110A) and causing the ultrasound-image-trained model (110A) to recognize a second feature region (200) shown in the ultrasound image (29B), to the display device (18) in a case in which the ultrasound image (29B) is displayed in a first display region (35A) of the display device (18).

15. The medical support device (24) according to claim 14,
wherein the second recognition result (128) includes second positional information (BB1, BB2) for specifying a position of the second feature region (200), second feature information (202A) for specifying a medical feature of the second feature region (200), and/or second part information (BB2, 202B) for specifying a part inside a body as a target of an examination via the ultrasound endoscope (16).

16. The medical support device (24) according to claim 15,
wherein the ultrasound-image-trained model (110A) includes a second trained model (110A1) to which the ultrasound image (29B) is input, and
the second trained model (110A1) generates the second positional information (BB1, BB2) based on the input ultrasound image (29B).

17. A medical support method comprising:
acquiring first image information (126) including a first optical image (29A) and an ultrasound image (29B), which are generated by performing imaging via an ultrasound endoscope (16), in a case in which the ultrasound endoscope (16) is connected to an image processing device (17) to which the ultrasound endoscope (16) and an optical endoscope (300) are connectable;
acquiring second image information (131) including a second optical image (329A), which is generated by performing imaging via the optical endoscope (300), in a case in which the optical endoscope (300) is connected to the image processing device (17);
outputting the first optical image (29A) and the ultrasound image (29B), which are included in the first image information (126), to a display device (18) in a case in which the ultrasound endoscope (29B) is connected to the image processing device (17); and
outputting the second optical image(329A), which is included in the second image information (131), and a first recognition result (139), which is obtained by inputting the second optical image (329A) to an optical-image-trained model (110B) and causing the optical-image-trained model (110B) to recognize a first feature region (250) shown in the second optical image (329A), to the display device (18) in a case in which the optical endoscope (300) is connected to the image processing device (17).
